# EUROPEAN PATENT APPLICATION

(11) **EP 4 300 102 A2**
(43) Date of publication of application: **03.01.2024**
(21) Application number: 23208532.4
(22) Date of filing: 06.09.2018
(51) Int. Cl.: G01N 33/569

(54) **IMPROVED IMMUNOTHERAPY**

(30) Priority: 08.09.2017 SE 1751091; 07.01.2018 SE 1850017
(62) Divisional of application: 18853717.9
(71) Applicant: Diamyd Medical AB, 111 56 Stockholm (SE)
(72) Inventor: LUDVIGSSON, Johnny, 581 83 Linköping (SE); CASAS, Rosaura, 581 83 Linköping (SE); HANNELIUS, Ulf, 111 56 Stockholm (SE); LINDQVIST, Anton, 111 56 Stockholm (SE); ESSEN-MÖLLER, Anders, 115 25 Stockholm (SE)
(74) Representative: Zacco Sweden AB

(57) **Abstract**

The present invention relates to a method for assessing the efficacy of an immunotherapy administered to a patient, said immunotherapy comprising administration of GAD, comprising the following steps: measuring at least one of GADA IgG subclass distribution; GADA levels; Distribution of cytokines secreted from lymphocytes; and Lymphocyte proliferation in presence of GAD or CD3/CD28 beads; in a first blood, plasma or serum sample obtained from said patient at a first point in time and in a second blood, plasma or serum sample obtained from said patient at a second, later, point in time; and comparing the so obtained measurements.

## Description

### Field of the invention

The present invention generally relates to the field of immunology and in particular immunotherapy. More particularly, the present invention pertains to the prevention and/or treatment of autoimmune diseases such as type 1 diabetes or autoimmune diabetes. The present invention provides biomarkers and administration regimens that are particularly useful in the prevention and/or treatment of such disease.

### Background

Type 1 diabetes (T1D) treatment consists of lifelong administration of insulin, a replacement therapy which does not satisfactorily prevent serious complications. Efforts to delay or halt disease progression have been ongoing for several decades, clinical intervention trials with recent-onset T1D patients have shown no or limited efficacy, which highlights the complexity of translation from animal models to human T1D. Immunomodulation with autoantigens could potentially constitute the most specific and safe treatment for T1D. Subcutaneous administration of glutamic acid decarboxylase (GAD)₆₅ formulated with aluminum hydroxide (GAD-alum) showed efficacy in preserving residual insulin secretion in children and adolescents with recent-onset T1D but subsequent phase II and phase III trials failed to reach their primary outcomes. However, significant efficacy was shown in pre-specified subgroups in the phase III study, and it has been shown that close administration of influenza vaccine might have influenced the study outcome. Thus, it is most probable that treatment with GAD-alum might be beneficial but has not been sufficiently effective.

In order to render the presentation of GAD₆₅ antigen to T cells in the lymph nodes more efficient than previously described, GAD-alum was administrated into lymph nodes to six patients participating in an open-label clinical trial, DIAGNODE. Results from these patients showed that preservation of C-peptide in the patients appeared to be similar to promising results observed in patients from other immune intervention trials.

### Summary of the invention

The present inventors have surprisingly found new biomarkers and combination of biomarkers that can be measured and analysed in patients receiving immunotherapy by administration of GAD to assess the efficacy of the immunotherapy. The present inventors have also identified the need in some patients receiving prior art immunotherapy to receive further administrations of the autoantigen GAD.

Thus, in a first aspect, the invention relates to a method for assessing the efficacy of an immunotherapy administered to a patient, said immunotherapy comprising administration of GAD, comprising the following steps:
- measuring at least one of
   - GADA IgG subclass distribution;
   - GADA levels;
   - Distribution of cytokines secreted from lymphocytes; and
   - Lymphocyte proliferation in presence of GAD or CD3/CD28 beads;
   in a first blood, plasma or serum sample obtained from said patient at a first point in time and in a second blood, plasma or serum sample obtained from said patient at a second, later, point in time;
- Comparing the so obtained measurements;
wherein an increased relative amount of IgG₂, IgG₃, and/or IgG₄, or decreased relative amount of IgG₁ in the GADA IgG subclass distribution; increased GADA levels; an increased relative amount of IL-13 and/or IL-5 or a decreased relative amount of IFNγ and/or TNFα in the distribution of cytokines secreted from lymphocytes; and/or reduced lymphocyte proliferation in the presence of GAD or CD3/CD28 beads; as measured in the second sample as compared to as measured in the first sample, is indicative of an effective immunotherapy.

In one aspect, the invention relates to a method for treatment or prevention of type 1 diabetes by means of immunotherapy, comprising the steps of
- administration of GAD to a subject;
- obtaining an assessment of the efficacy of the immunotherapy by a method according to the above; and
- adjusting the dosage and/or administration route of GAD based on said assessment.

In one aspect, the invention relates to a method for treatment or prevention of type 1 diabetes in a subject by means of immunotherapy, comprising the steps of
- administration of vitamin D commencing at day 1 and continuing for 3 to 6 months;
- three administrations of GAD into a lymph node at days 30, 60 and 90, respectively; and
- a fourth administration of GAD into a lymph node at a time between 12 and 18 months after day 1.

The invention also relates to GAD for use in the methods according to the invention and to the use of GAD in the manufacture of a pharmaceutical composition for use in the methods according to the invention.

Certain embodiments of the invention are further described below and in the appended claims.

### Description of the drawings

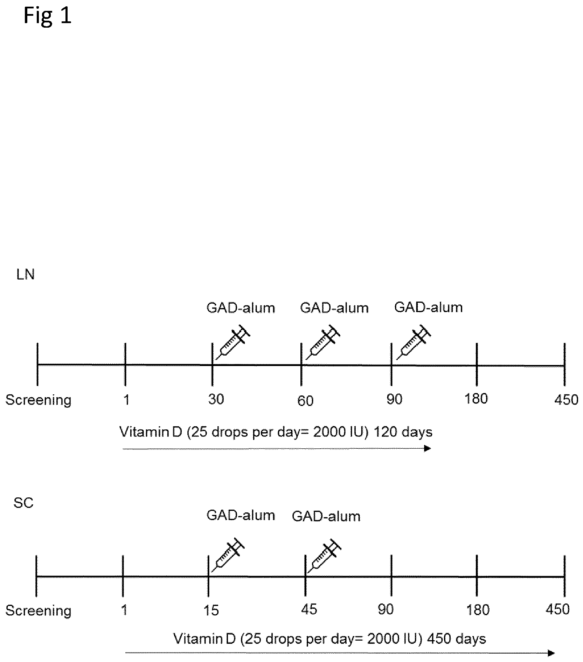

Fig. 1: Overview of the GAD-alum treatment. (a) Type 1 diabetic patients (n=6) received a first injection of GAD-alum (4µg) into the lymph-nodes (LN), followed by two booster injections one month apart. A control group of patients who participated in a another study (n=6) have received a first subcutaneous (SC) GAD-alum dose (20 µg) followed by a second injection after one month. All patients received in parallel Vitamin D (Calciferol), during the first 120 days the LN patients and for 450 days the SC group.

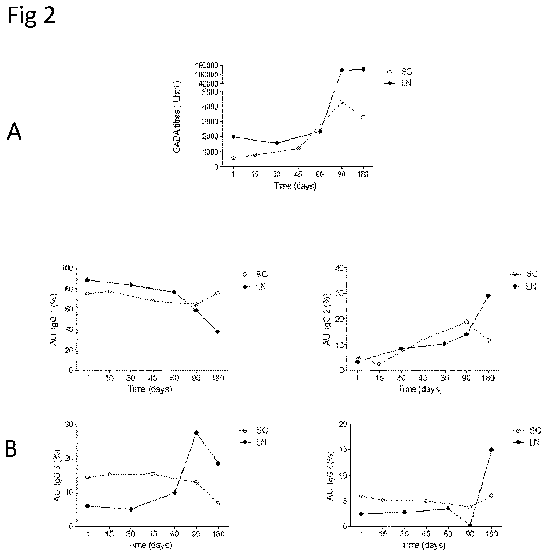

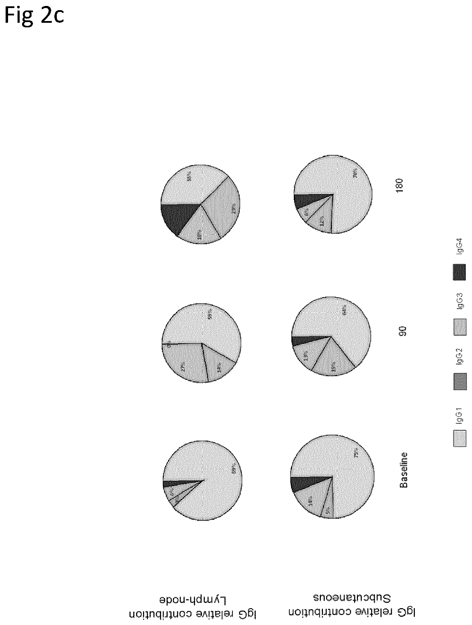

Fig 2: a) Mean values of GADA titres for patients who received GAD-alum injections into the lymphnodes (LN, n=6) or subcutaneously (SC, n=6). (b) Change of the frequency (%) of IgG₁, IgG₂, IgG₃, IgG₄ GADA subclasses. Frequencies were calculated with respect to the combined sum of the 4 subclasses in each sample (i.e. total IgG). The median percentage respect to the total IgG is shown for each respective subclass. (c) GADA subclass relative contribution at baseline, 90 and 180 days for LN and SC groups.

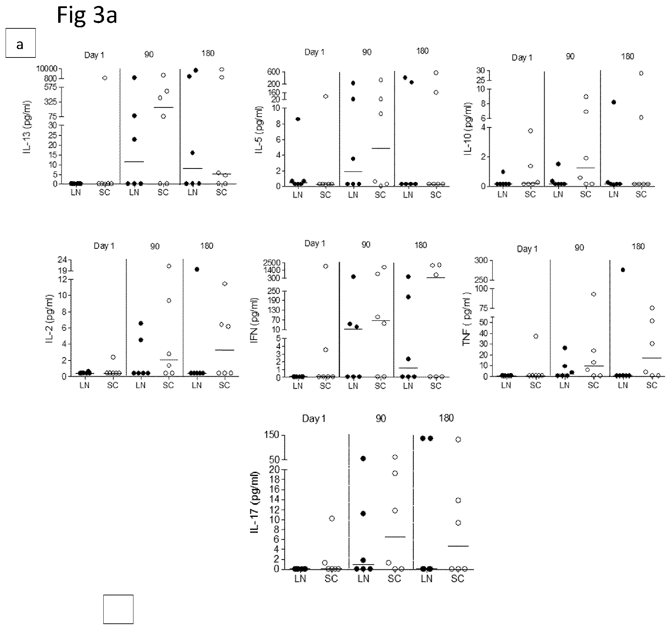

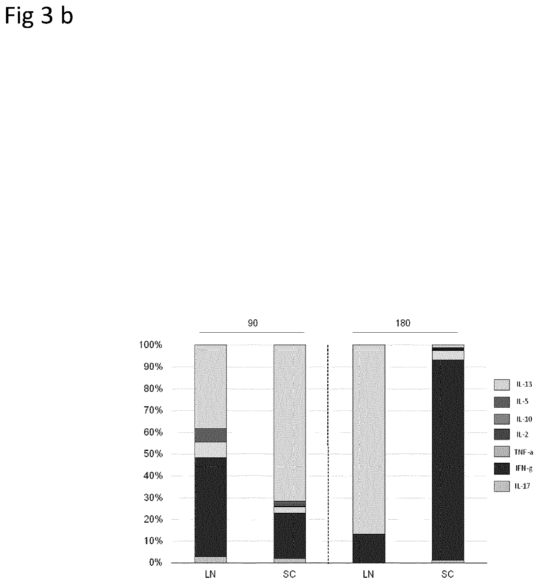

Fig 3: GAD65-sinduced cytokine secretion upon in vitro PMBC stimulation. Patients received GAD-alum injections into the lymph-nodes (LN, n=6) or subcutaneously (SC, n=6). (a) Median levels of IL-13, IL-5, IL-10, IL-2, IL-17, TNF-α and IFN-γ (pg/ml) at baseline, 90 and 180 days for LN (black circles) and SC (white circles) patients were detected by Luminex in supernatants collected after 7 days culture in presence of medium or GAD65 (5µg/ml). GAD65-induced cytokine secretion is given after subtraction of spontaneous secretion from each individual. (b) Relative contribution (%) of the cytokines in the lymph-node patients (LN) and subcutaneous group (SC) at 90 and 180 days.
Fig: 4 Proliferative responses to GAD65 and CD3/CD28 beads. Patients received GAD-alum injections into the lymph-nodes (LN, n=6) or subcutaneously (SC, n=6). PBMC were culture for 3 days with GAD₆₅ (5µg/ml), CD3/CD28 beads or medium, and thereafter cells were pulsed with [³H] thymidine and harvested. Proliferation is expressed as stimulation index (SI), calculated from the mean of triplicates divided by the mean of triplicates with medium alone.

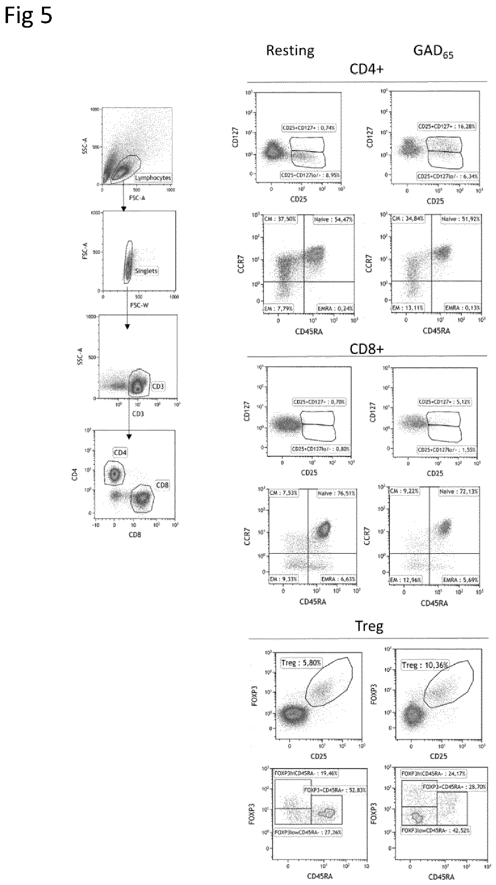

Fig 5: T cell activation induced by GAD₆₅. PBMCs from baseline (day 1), 90 and 180 days from patients who received GAD-alum into the lymph node (n=6, LN, black circles) and subcutaneous (n=6, SC, white circles) were stimulated for 7 days with GAD₆₅ (5µg/ml) or medium. (a) Percentage of GAD₆₅-activated CD4⁺CD25⁺CD127⁺ T cells and (b) CD8⁺ CD25⁺CD127⁺ T cells. (c) Mean percentage of CD4⁺ CD25⁺CD127^{lo/-} FOXP3⁺ (Treg) in resting samples (medium alone) and (d) induced by GAD₆₅ stimulation. Mean percentage of FOXP3^{lo}CD45RA non-suppressor regulatory T cells in (e) resting samples (medium alone) and (f) GADss-stimulated samples.

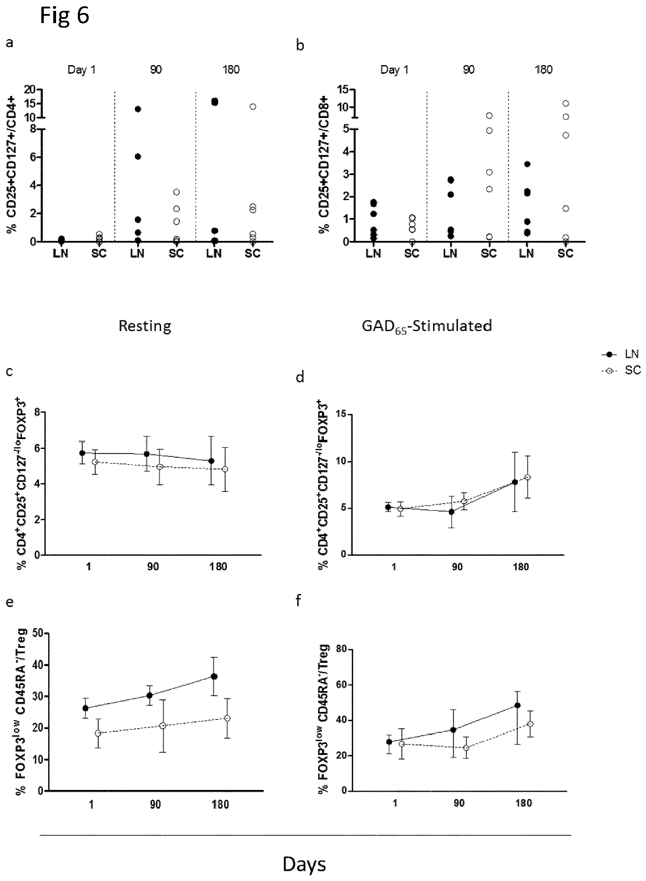

Fig 6: Change of differentiation state (%) of CD4⁺ and CD8⁺T cells. PBMCs from baseline (day 1), 90 and 180 days from patients who received GAD-alum into the lymph node (n=6, LN, black circles) and subcutaneous (n=6, SC, white circles) were cultured for 7 days with GAD₆₅ (5µg/ml) or medium. GAD₆₅ induced changes of (a) Näive (T_{N}, CD45RA⁺CCR7⁺), (b) Central memory (T_{CM}, CD45RA⁻CCR7⁺), (c) effector memory (T_{EM}, CD45RA⁻CCR7₋) and (d) terminally differentiated effector memory (T_{EMRA}, CD45RA⁺CCR7⁻) CD4 T cells. GAD-alum induced change of (e) näive (T_{N}, CD45RA⁺CCR7⁺), (f) central memory (T_{CM}, CD45RA⁻CCR7⁺) (g) Percentage effector memory (T_{EM}, CD45RA⁻CCR7₋) and (h) terminally differentiated effector memory (T_{EMRA}, CD45RA⁺CCR7⁻) CD8 T cells. Lines represent mean trend.

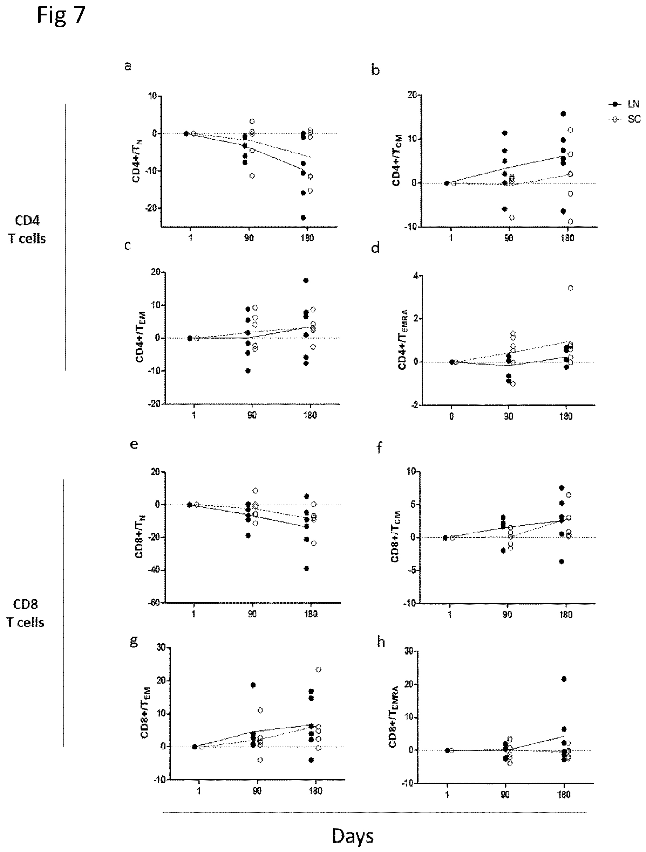

Fig 7: Representative flow cytometry analysis from one patient who received lymph node injection of GAD-alum. Sample was collected at 180 days, and PBMC were cultured for 7 days in the presence of GAD₆₅ (5µg/ml) or medium. The percentages of CD4⁺, CD8⁺ T cells and Tregs were assessed in resting (medium alone) and GAD₆₅-stimulated samples.

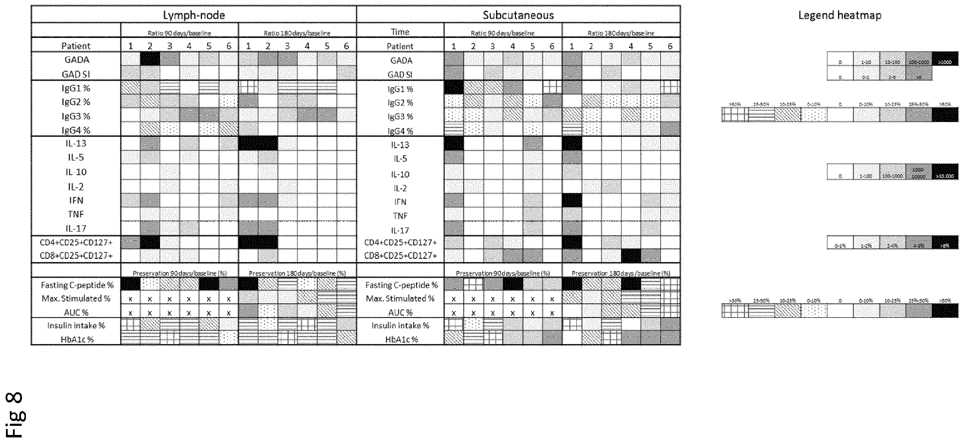

Fig 8: Heatmap was created to represent the immunological changes induced by GAD-alum treatment. Changes were calculated as the ratio of the values at 90 and 180 days respect to the baseline. Patients received GAD-alum injections into the lymph-nodes (LN, n=6) or subcutaneously (SC, n=6), and they were stratified from left to right according their clinical outcome at 180 days.

Clinical variables are expressed as percentage of change from baseline (%). At 90 days, Max.stimulated and AUC c-peptide were not calculated as meal tolerance tests no performed, and are represented by "x". The greyscales illustrate the post-treatment increase of immunological variables in relation to baseline values.

### Definitions

All terms and expressions as used herein are intended to have the meaning given to them by the person skilled in the art at the filing date of the present application, unless any other expression is evident from the context of this disclosure. However, for the sake of clarity, some terms and expressions are explicitly defined below.

An "autoantibody" is an antibody that reacts with autoantigens of the organism that produced them.

An "autoantigen" or "self-antigen" is an endogenous tissue constituent that has the ability to interact with autoantibodies and cause an immune response. A "beta cell autoantigen" is an autoantigen originating from pancreatic beta cells.

As used herein, "coadministration" refers to administering the compounds of the regimen of the present invention so that their dosing regimens overlap. They do not need to be administered at the same time.

The term "cyclooxygenase inhibitors", or "cox inhibitor", relates to compounds that combine with cyclooxygenase and thereby prevent its substrate-enzyme combination with arachidonic acid and the formation of eicosanoids, prostaglandins, and thromboxanes. A subgroup of the cyclooxygenase inhibitors is the cyclooxygenase-2 inhibitors, which have specificity for cyclooxygenase-2.

"Day 1" of a method of treatment is considered the day on which the first administration of a compound included in the treatment regimen is performed. In the context of the present invention, it is in general the first administration of vitamin D or GAD to a patient to initiate a method of treatment according to the invention.

As used herein, the "distribution" of a class of compounds, such as cytokines or antibodies, relates to the relative amounts or concentrations of the specific compounds belonging to that class as compared to the total amount or concentration of all compounds belonging to that class.

An "epitope" is the surface portion of an antigen capable of eliciting an immune response and of combining with the antibody produced to counter that response, or a Tcell receptor.

"GAD" relates to glutamic acid decarboxylase and "GAD-alum" to glutamic acid decarboxylase formulated with aluminum hydroxide. GAD65 is the 65 kDa form of GAD.

The term "gamma-amino butyric acid analogs" includes vigabatrin and baclofen.

The term "required insulin dose" denotes the average daily insulin dose required by a diabetes patient to control his or her blood sugar levels.

The term "Vitamin D" includes vitamin D₂ and vitamin D₃. "Vitamin D analogs" include without prejudice Ergocalciferol, Dihydrotachysterol, Alfacalcidol, Calcitriol, Colecalciferol, and Calcifediol, and combinations thereof, as well as any other vitamin D analog classified in group A11CC of the Anatomical Therapeutic Chemical Classification System.

The term "TNF alpha inhibitor" relates to compounds that inhibits the action of Tumour Necrosis Factor alpha (TNF alpha), and includes adalimumab, certolizumab, etanercept, golimumab, infliximab, as well as any other compound classified in group L04AB of the Anatomical Therapeutic Chemical Classification System.

Expressions using the singular "a", "an" and the like shall be construed as including the plural.

### Abbreviations

GABA: gamma-amino butyric acid
GADA: Glutamic Acid Decarboxylase Autoantibodies
LN: lymph-node
PBMC: Peripheral blood mononuclear cell
SC: subcutaneous
Th: T helper
T1D: type 1 diabetes

### Detailed description of the invention

The present inventors have studied how a number of biomarkers correlate with clinical outcome of immunotherapy using GAD. Our results described in the experimental section identify a plurality of biomarkers that can be used to assess the efficacy of a therapy using GAD. No specific immune signatures before treatment were identified among the biomarkers included in this study, but patients with improved metabolic outcome, i.e. lower HbA1c and insulin needs, and better preservation of C-peptide secretion seemed to have some common immune correlates.

A major pending question in T1D intervention trials is the identification of the mechanism behind treatment as well as the definition of pre- and post-treatment immune signatures. Based on the general consensus that T1D is due to the lack of tolerance and the involvement of autoreactive T cells, it has been expected that efficacy of immunotherapy with autoantigens should be accompanied by the induction of tolerance and loss of the immune response to the autoantigen in question. Here we observe that individuals with better clinical outcome after LN injections had an immune response characterized by rise of GADA, reduction of proliferation, and induction of predominant Th2-like responses, especially increase of IL-13, supported by the shift of IgG subclasses and GAD-induced cytokine secretion.

Although the direction of the immune responses to GAD₆₅ were Th2-skewed in the LN group, there were however large inter-individual differences. Strikingly, GAD₆₅-induced cytokines were detected in the LN group after 180 days solely in patients with a best clinical outcome.

It was particularly interesting that patients displaying a better clinical outcome were characterized by GAD-induced T-cell responses deviated towards a Th2 cytokine profile, both after SC and LN treatment. T-cell responses in the LN patients did not show a tolerogenic deviation, but rather a Th2-associated profile, and some Th1 cytokines were also produced. However, Th2 cytokines were more dominant in the responders in the LN group than in the SC patients. We have previously shown that the cytokine secretion was characterized by a broad cytokine profile short after SC injection of 2 doses of GAD-alum, and cytokine secretion tended to switch towards a more predominant Th2-associated profile over time. In that study, administration of further doses increased the secretion levels, but did not affect the quality of the cytokine response, and cytokine profile was similar in patients receiving 2 or 4 doses of GAD-alum. Thus, predominant secretion of IL-13 after the third LN injection cannot be explained by an extra dose but rather by the administration route. Intralymphatic administration delivers more antigen to the site of immune response induction and the difference in antigen dose available for stimulation of antigen specific T cells may also lead to increased Th2 response. The adjuvant aluminum effect is associated with the induction of Th2 responses, and preferentially induce humoral rather than cellular immune responses, thus alum has been the adjuvant of choice to minimize the possibility of promoting-mediated b-cell destruction with GAD₆₅.

A number of biomarkers of clinical outcome have thus been identified.

Individual immunological changes induced by GAD-alum treatment were calculated as the ratio of GAD₆₅-induced immune responses at 90 and 180 days respect to specific immune responses pre-treatment, and patients were stratified for each treatment according to their metabolic and c-peptide preservation (Figure 8).

Baseline immunological parameters did not show any pre-treatment feature that seemed to be related to the clinical outcome.

Representation of induced changes of antigen-induced cytokine secretion post treatment showed that Th1 and Th2 associated cytokines were induced by GAD₆₅ stimulation after 90 days in most of the patients, independently of the administration route. However, changes on GAD₆₅-induced cytokines at 180 days were detectable in the LN patients with the best clinical response (n=3), i.e., lower HbAc1, decreased insulin intake and best preservation of c-peptide secretion. Intriguingly, activated CD4 cells but no CD8 were observed in these three patients. Reduction of IgG₁ and enhancement of IgG₂ and IgG₄ was most pronounced in the patient with the best clinical response (patient 1), and this patient accounted for the observed increase of IgG₄ in the LN group (Figure 8).

GAD₆₅ induced cytokine secretion in the patient with best clinical response in the SC group (patient 1) resembled that observed in the LN patients with best response, but in the SC treated patient IgG1 was the predominant GADA subclass, and both activated CD4 and CD8 cells were detected.

Calculation of the ratio of Th2 (IL-13 and IL-5)/Th1 (IFN-y and TNF-α) cytokines at 180 days for the better responders in both groups (n=2 LN and n=1 in SC) revealed that Th2 response was three times stronger in LN patients than in the SC group (ratio: 6.44 LN vs 2.24 SC).

Thus, in one aspect, the present invention relates to a method method for assessing the efficacy of an immunotherapy administered to a patient, said immunotherapy comprising administration of GAD, comprising the following steps:
- measuring at least one of
   - GADA IgG subclass distribution;
   - GADA levels;
   - Distribution of cytokines secreted from lymphocytes; and
   - Lymphocyte proliferation in presence of GAD or CD3/CD28 beads;

in a first blood, plasma or serum sample obtained from said patient at a first point in time and in a second blood, plasma or serum sample obtained from said patient at a second, later, point in time;
   - Comparing the so obtained measurements;
wherein an increased relative amount of IgG₂, IgG₃, and/or IgG₄, or decreased relative amount of IgG₁ in the GADA IgG subclass distribution; increased GADA levels; an increased relative amount of IL-13 and/or IL-5 or a decreased relative amount of IFNy and/or TNFα in the distribution of cytokines secreted from lymphocytes; and/or reduced lymphocyte proliferation in the presence of GAD or CD3/CD28 beads; as measured in the second sample as compared to as measured in the first sample, is indicative of an effective immunotherapy.

The designation of samples as a "first" and a "second" sample is intended to only define the relation between the samples as being obtained or having been obtained at a first, prior, time and a second, later, time, and does not exclude that further samples may have been obtained, and optionally analysed, prior to the first sample or between obtaining the first and the second sample.

In one embodiment at least two, at least three, or four of
- GADA IgG subclass distribution;
- GADA levels;
- Distribution of cytokines secreted from lymphocytes; and
- Lymphocyte proliferation;
are measured in said first and second blood, plasma or serum sample.

In one embodiment the first sample is obtained before or at commencement of the immunotherapy, or 80-100, such as 90, days after commencement of the immunotherapy.

In one embodiment the second sample is obtained at 160-200 days, such as 180 days; or at 12, 15, 24, 30 or 36 months after commencement of the immunotherapy.

In one embodiment the second sample is obtained 160-200 days, such as 180 days; or at 12, 15, 24, 30 or 36 months after the first sample is obtained.

In one embodiment the immunotherapy comprises daily administration of vitamin D commencing at day 1, and intralymphatic injection of GAD at days 30, 60 and 90.

In one embodiment the administration of vitamin D commencing at day 1 extends for 3 months or more, such as 4, 5, or 6 months.

In one embodiment GADA IgG subclass distribution is measured in said first and second samples and the so obtained measurements are compared, and wherein an increased relative amount of IgG₂ in the GADA IgG subclass distribution, as measured in the second sample as compared to as measured in the first sample, is indicative of an effective immunotherapy.

In one embodiment GADA IgG subclass distribution is measured in said first and second samples and the so obtained measurements are compared, and wherein an increased relative amount of IgG₃ in the GADA IgG subclass distribution, as measured in the second sample as compared to as measured in the first sample, is indicative of an effective immunotherapy.

In one embodiment GADA IgG subclass distribution is measured in said first and second samples and the so obtained measurements are compared, and wherein an increased relative amount of IgG₄ in the GADA IgG subclass distribution, as measured in the second sample as compared to as measured in the first sample, is indicative of an effective immunotherapy.

In one embodiment GADA IgG subclass distribution is measured in said first and second samples and the so obtained measurements are compared, and wherein a decreased relative amount of IgG₁ in the GADA IgG subclass distribution, as measured in the second sample as compared to as measured in the first sample, is indicative of an effective immunotherapy.

In one embodiment GADA IgG subclass distribution is measured in said first and second samples and the so obtained measurements are compared, and wherein an increased amount of IgG₄ relative to the amount of IgG₁ in the GADA IgG subclass distribution, as measured in the second sample as compared to as measured in the first sample, is indicative of an effective immunotherapy.

In one embodiment GADA IgG subclass distribution is measured in said first and second samples and the so obtained measurements are compared, and wherein an increased amount of IgG₂ relative to the amount of IgG₁ in the GADA IgG subclass distribution, as measured in the second sample as compared to as measured in the first sample, is indicative of an effective immunotherapy.

In one embodiment GADA IgG subclass distribution is measured in said first and second samples and the so obtained measurements are compared, and wherein an increased amount of IgG₁ relative to the amount of IgG₁ in the GADA IgG subclass distribution, as measured in the second sample as compared to as measured in the first sample, is indicative of an effective immunotherapy.

In one embodiment GADA levels are measured in said first and second samples and the so obtained measurements are compared, wherein increased GADA levels, as measured in the second sample as compared to as measured in the first sample, are indicative of an effective immunotherapy.

In one embodiment distribution of cytokines secreted from lymphocytes is measured in said first and second samples and the so obtained measurements are compared, wherein increased relative amount of IL-13 and/or IL-5, as measured in the second sample as compared to as measured in the first sample, is indicative of an effective immunotherapy.

In one embodiment distribution of cytokines secreted from lymphocytes is measured in said first and second samples and the so obtained measurements are compared, wherein decreased relative amount of IFNy and/or TNFα, as measured in the second sample as compared to as measured in the first sample, is indicative of an effective immunotherapy.

In one embodiment distribution of cytokines secreted from lymphocytes is measured in said first and second samples and the so obtained measurements are compared, wherein an increased amount of IL-13 relative to the amount of IFNy, as measured in the second sample as compared to as measured in the first sample, is indicative of an effective immunotherapy.

In one embodiment distribution of cytokines secreted from lymphocytes is measured in said first and second samples and the so obtained measurements are compared, wherein an increased amount of IL-5 relative to the amount of IFNy, as measured in the second sample as compared to as measured in the first sample, is indicative of an effective immunotherapy.

In one embodiment distribution of cytokines secreted from lymphocytes is measured in said first and second samples and the so obtained measurements are compared, wherein an increased amount of IL-13 relative to the amount of TNFα, as measured in the second sample as compared to as measured in the first sample, is indicative of an effective immunotherapy.

In one embodiment distribution of cytokines secreted from lymphocytes is measured in said first and second samples and the so obtained measurements are compared, wherein an increased amount of IL-5 relative to the amount of TNFα, as measured in the second sample as compared to as measured in the first sample, is indicative of an effective immunotherapy.

In one embodiment lymphocyte proliferation in presence of GAD or CD3/CD28 beads is measured in said first and second samples and the so obtained measurements are compared, wherein reduced lymphocyte proliferation in the presence of GAD or CD3/CD28 beads, as measured in the second sample as compared to as measured in the first sample, is indicative of an effective immunotherapy.

In one embodiment said immunotherapy comprises administration of GAD by means of intralymphatic injection, including injection into a lymph node, intradermal injection, subcutaneous injection, or oral administration.

In a further aspect, the invention relates to a method for treatment or prevention of type 1 diabetes by means of immunotherapy, comprising the steps of
- administration of GAD to a subject;
- obtaining an assessment of the efficacy of the immunotherapy by a method according to the above; and
- adjusting the dosage and/or administration route of GAD based on said assessment.

In one embodiment the adjustment of the dosage of GAD includes a further administration of GAD by injection into a lymph node, if the comparison of the obtained measurements is not indicative of an effective immunotherapy.

In one embodiment, the method comprises
- administration of vitamin D commencing at day 1 and continuing for 3 to 6 months;
- three administrations of GAD into a lymph node of the subject at days 30, 60 and 90, respectively;
- obtaining an assessment of the efficacy of the immunotherapy by a method according to the above;
and wherein if the comparison of the obtained measurements is not indicative of an effective immunotherapy, then the adjustment of the dosage of GAD includes a fourth administration of GAD into a lymph node at a time between 12 and 18 months after day 1.

Furthermore, while administration of GAD-alum directly into a lymph node has been quite successful, the present inventors have found that the production of insulin in some treated patients decreased from 15 months to 30 months from commencement of the treatment regimen, as measured by stimulated C-peptide AUC (down 32%). However, HbA1c and insulin use were lower than at the start of treatment (down 15% and 22%, respectively).

The present inventors have therefore found that further administration of GAD-alum into a lymph node can be beneficial. For example, a fourth dose of GAD-alum into a lymph node could be made between 12 and 18 months from treatment start, such as at 15 months.

Thus, in one aspect the invention relates to method for treatment or prevention of type 1 diabetes in a subject by means of immunotherapy, comprising the steps of
- administration of vitamin D commencing at day 1 and continuing for 3 to 6 months;
- three administrations of GAD into a lymph node at days 30, 60 and 90, respectively; and
- a fourth administration of GAD into a lymph node at a time between 12 and 18 months after day 1.

The fourth administration of GAD may be performed after performing an assessment of the efficacy of the immunotherapy, according to the invention as described above. Analogously, further administrations of GAD may be performed after performing an assessment of the efficacy of the immunotherapy, according to the invention as described above, at any given time during the immunotherapy provided to a subject.

In one embodiment, the fourth administration is performed if HbA1c and required insulin dose have gone up at 12 to 18 months after day 1 as compared to the levels observed at 5 to 6 months after first GAD-alum injection, and optionally if the IFN/IL-13 ratio has not decreased between the said observations.

In one embodiment, a further administration of GAD-alum is performed at 30 months after day 1 if HbA1c and required insulin dose have gone up at 30 months as compared to the levels observed at 15 months after first GAD-alum injection, and optionally if the IFN/IL-13 ratio has not decreased between the said observations.

In one embodiment, the treatment method according to the invention comprises at least three administrations of GAD into a lymph node of the subject at days 30, 60 and 90, respectively, and wherein at least one further administration into a lymph node is performed if HbA1c levels and/or required insulin dose for the subject at a given time have increased as compared to levels observed 6-24 months prior to said given time.

In one embodiment, the treatment method according to the invention comprises at least three administrations of GAD into a lymph node of the subject at days 30, 60 and 90, respectively, and wherein at least one further administration into a lymph node is performed if the relative amount of IL-13 and/or IL-5 in the distribution of cytokines secreted from lymphocytes is unchanged or decreased at a given time as compared to levels observed 6-24 months prior to said given time.

In one embodiment, the treatment method according to the invention comprises at least three administrations of GAD into a lymph node of the subject at days 30, 60 and 90, respectively, and wherein at least one further administration into a lymph node is performed if the ratio IFNy/IL-13 in the distribution of cytokines secreted from lymphocytes is unchanged or increased at a given time as compared to a the same ratio observed 6-24 months prior to said given time.

In one embodiment, the treatment method according to the invention comprises at least three administrations of GAD into a lymph node of the subject at days 30, 60 and 90, respectively, and wherein at least one further administration into a lymph node is performed if the ratio of IgG₁/IgG₄ in the population of GAD specific antibodies is unchanged or increased at a given time as compared to the same ratio observed 6-24 months prior to said given time.

In one embodiment, the treatment method according to the invention comprises daily administration of vitamin D commencing 0-90 days prior to a fourth or any further administration of GAD into a lymph node.

In one embodiment, the treatment method according to the invention, vitamin D is administered at a dose of 2000 IU per day.

In one embodiment, the treatment method according to the invention, vitamin D is administered for 4 months.

In one embodiment, the treatment method according to the invention, GAD is administered in the form of alum-formulated GAD.

In one aspect, the present invention provides GAD for use in a method for treatment according to the invention.

In one aspect, the present invention provides the use of GAD in the manufacture of a pharmaceutical composition for use in a method for treatment according to the invention.

The methods of treatment according to the present invention may utilize compositions, methods, and administration regimens as generally described in WO2015/187087, the disclosure of which is incorporated herein by reference, as applied to treatment of Type 1 Diabetes using the beta cell autoantigen GAD. For full disclosure, such methods are described below.

In one aspect, the present invention utilizes a method for prevention and/or treatment of an autoimmune disease, comprising administering a composition, said composition comprising at least one beta cell autoantigen, to a subject having a serum vitamin-D level above 50 nanomole/liter. Each of the at least two molecules may thus influence the reaction of the adaptive immune cell to which the antigens are presented.

The subject may have a serum D-vitamin level between 50-150 nanomole/liter, such as 60-100 nanomole/liter, 75-100 nanomole/liter or 100-150 nanomole/liter.

The method may comprise a pretreatment of the subject to adjust the serum vitamin-D level, and such pretreatment may comprise administration of vitamin-D and/or vitamin-D analogs, and/or exposure to UVB-radiation, preferably for between 7 to 90 days before administration of the composition comprising at least one beta cell autoantigen to said subject.

The method may further comprise administration of vitamin-D and/or vitamin-D analogs in an amount of 7000-70000 IU/week for 3-48 months.

The beta cell autoantigen is GAD, as defined above.

The method may further comprise administration of a cyclooxygenase inhibitor, as discussed below under the heading "Cyclooxygenase inhibitors".

The method may further comprise administration of a CTLA4 compound, as discussed below under the heading "CTLA4 compounds".

The method may further comprise administration of a TNFalpha inhibitor, as discussed below under the heading "TNFalpha inhibitors".

The present invention provides a method for the prevention and/or treatment of an autoimmune disease in an individual in need thereof, the method comprising administering to said individual:
a) for specific antigen tolerization purposes, at least one autoantigen or fragments thereof; or nucleic acids, plasmids or vectors coding for such molecules related to at least one of the autoimmune and inflammatory diseases as listed above. In one embodiment, autoantigen is administered when serum vitamin D levels are between 50 and 150nM/l, more preferably between 75 and 100 nM/l and most preferably between 100 -150nM/I; and
b) for interfering with APCs ability to mature, administering to said individual at least one IL-10 inducing compound selected from the group consisting of vitamin-D, vitamin-D analogs, gamma-amino butyric acid, gamma-amino butyric acid analogs and tyrosine kinase inhibitors; as listed above. In one embodiment the IL-10 induction is enhanced or accomplished by use of UVB-light exposure; and
c) for interfering with the immune system's ability to activate naive TCs and BCs and recall responses from activated and memory lymphocytes, administering a compound such as a NSAID compound; a CTLA-4 compound; or a TNFalpha inhibitor; as listed above.

The invention discloses a method for treatment of autoimmune disease, such as T1D and autoimmune diabetes, the method comprising administering to a subject with said disease:
(a) a course of Vit D for enforcing the ability of antigen presenting dendritric cells to present antigen peptides to the immune system in a tolerizing manner;
b) an autoantigen, such as GAD65, formulated in a pharmaceutical carrier administered in an amount sufficient to restore or induce tolerance to the autoantigen; and optionally
c) a therapeutic dose of an anti-inflammatory compound, for example a cyclooxygenase inhibitor such as Ibuprofen, or a more pronounced cox-2 or cox-1 inhibitor.

The course of Vit D preferably starts 15 to 90 days prior to administration of autoantigen, or 7 to 90 days prior to administration of autoantigen, and is given in liquid or tablet form in doses corresponding to 7000 to 70000 iu per week for a period of 3 to 48 months.

The pretreatment with vitamin D aims to elevate the treated subject's serum levels of vitamin D to above about 50 nanomole/liter, or above 60, 75, or 100 nanomole/liter. The pretreatment may be dispensed with if the subject already has serum levels of vitamin D at these levels.

In another embodiment of the invention the serum concentration of Vit D can be enhanced by means of phototherapy. In this case subjects will be exposed to ultraviolet B radiation preferably between 10-120 minutes daily for 15 to 90 days prior to administration of autoantigen. The phototherapy should continue for a period of 3 to 48 months.

Preferred doses of the autoantigen is between two and four administrations, at least two weeks apart, more preferably one month apart, of each between 10 and 200 µg antigen if given by injection. If administered orally the preferred doses are between 500mg and 5g daily for a period of between three months and 48 months. Ibuprofen is preferably administered in daily doses of 100 to 800mg for a period of 60 to 150 days during which period administration of autoantigen takes place.

The autoantigen can be administered by intralymphatic injection, i.e. injection directly into a lymph node. The anti-inflammatory compound and the autoantigen can be administered in/with a pharmaceutically acceptable carrier, excipient or diluent.

In one embodiment of the composition containing the beta cell autoantigen is administered 1-4 weeks apart, such as 2-4 weeks apart or 2 weeks apart, in an initial treatment period of 3 to 4 months, and optionally 2-3 months apart in a continued treatment period of 6-9 months.

In one embodiment, the amount of beta cell autoantigen is increased from 1-5 µg per administration at the beginning of the treatment period to about 40-100 µg per administration in the final administrations.

In one embodiment of this invention administration of autoantigen is made directly into the lymph nodes or into the lymphatic system to allow resident APCs to present antigen peptides to the immune system. If administration of autoantigens is made directly into a lymph node or into the lymphatic system, the dose will preferably be between 1 and 15 µg per autoantigen, more preferred between 2 and 10 µg per autoantigen or 2 to 5 µg per autoantigen. Formulation in alum is preferred.

According to certain embodiments, the at least one autoantigen is administered intrainguinal, intralymph node or intralymphatic. In some embodiments, the volume for intra-inguinal injection of the antigens is between 0.05 and 0.2 ml, more preferred between 0.05 and 0.15 ml.

According to certain embodiments, where the at least one antigen is administered by intralymphnode or intralymphatic injection a preferred dosage is between 1-15ug, more preferred between 2-10, and most preferred between 2-5ug per injection and autoantigen used, such administrations taking place at least 2 times, more preferred at least 3 times and most preferred at least 4 times, at least 14 days apart, more preferably at least 30 days apart.

According to certain embodiments, where the at least one antigen is administered intravenously, at least 100-10000 ug of each antigen is administered per treatment occasion at least twice, at least one week apart..

According to certain embodiments, where the at least one antigen is administered orally, at least 0.5-5g of each antigen is administered per treatment occasion at least once every week.

According to certain embodiments, the at least one antigen is formulated separately or together with the other antigens as the case may be, in alum, saline or human serum albumin.

According to certain embodiments, the at least one pharmaceutical composition comprises at least two antigens on the same carrier particle. According to certain other embodiments, the at least one pharmaceutical Composition comprises at least two antigens on different carrier particles. According to certain embodiments, the at least two antigens are administered separately and at different times and frequencies, regimens and formulations that are suitable for the specific antigens. More preferably the at least two antigens are formulated in the same pharmaceutical Composition and therefore administered simultaneously.

In some embodiments the at least one antigen is formulated in an adjuvant such as alum. In more particular embodiments the at least one antigen is formulated in saline or human serum albumin.

According to certain embodiments, the at least one antigen and the at least one IL-10 inducing compound are administered simultaneously. According to certain embodiments, the at least one antigen and the at least one IL-10 inducing compound are administered separately.

According to certain embodiments, the at least one IL-10 inducing compound is administered simultaneously with the at least one antigen. According to certain embodiments, the administration of the at least one IL-10 inducing compound commences between 1-14 days prior to the first administration of the at least one antigen. According to certain embodiments, the administration of the at least one IL-10 inducing compound commences at least 2 preferably at least 10 weeks, prior to the first administration of the at least one autoantigen.

According to certain embodiments that include treatment periods with Vitamin D, between 500 and 10000 IU, more preferably between 1000 and 3000 IU of vitamin D, such as Vitamin D3, are administered per day.

According to certain embodiments that include pretreatment periods with Vitamin D, between 7,000 - 100,000 IU of Vitamin D, such as Vitamin D3 are administered per week, prior to first administration of the at least one antigen, and as a maintenance dose of 500-2000 IU per day thereafter.

In some embodiments the treatment period of Vitamin D is between 60 and 420 days after 1^{st} administration of antigen.

The compound that reduces the immune system's ability to activate naive TCs and BCs and recall responses from activated and memory lymphocytes, such as a NSAID compound; a CTLA-4 compound; or a TNFα inhibitor may be administered simultaneously or separately with the at least one antigen and/or the at least one IL-10 inducing compound.

In some embodiments,the compound that reduces the immune system's ability to activate naive TCs and BCs and recall responses from activated and memory lymphocytes is a NSAID compound, such as a COX-inhibitor.

According to certain embodiments, the treatment period with NSAIDs starts at least 2 weeks prior to first administration of the at least one antigen.

According to certain embodiments, when the COX inhibitor is Ibuprofen, at least one 400 to 1000 mg doses are administered per day during the NSAID treatment period.

The NSAID treatment period is at least between 4 and 14 weeks, more preferred between 4 and 8 weeks

In the present methods, the anti-inflammatory compound should be administered orally or by injection.

In some embodiments, the compound that reduces the immune system's ability to activate naive TCs and BCs and recall responses from activated and memory lymphocytes is a TNFα inhibitor.

Blocking TNFalpha reduces the activation state of the immune response and decreases the activity of DCs and other immunocytes. Further, because TNFalpha disrupts FSC and GC architecture in lymphoid tissue and impairs B cell functions, the activation of antigen-specific effector T cells and autoantibody production is reduced. Recent diabetes preclinical data show that beta cell antigen delivered by quiescent DCs can induce peripheral T cell unresponsiveness, down-modulate ongoing beta cell destruction and arrest beta cell destruction. Therefore, under a combined autoantigen - TNFalpha inhibitor therapy autoantibody levels and effector T cell activity and numbers will be relatively reduced while the number of autoantigen-specific Tregs number and function will be at least maintained maintained. It takes months to modify these immunologic processes and document these effects, but during this time, due to its acute beta-cell-protective and metabolic effects, TNFalpha inhibition will also preserve beta cells directly. Because GAD65 is known to be one of the primary autoantigens in T1DM, this approach produces a deviation of the diabetes autoimmune response towards a regulatory phenotype of sufficient critical mass to lead to a significant and prolonged effect on beta cell preservation.

Thus, in one aspect of the present invention, a TNF-alpha inhibitor not limited to for example infliximab, adalimumab, golimumab, and etanercept, is used as an anti-inflammatory compound. A preferred dose when etanercept is used is between 0.2 and 1mg/kg SQ, once or twice per week for a period of between 2 to 9 months.

According to certain embodiments, when the TNFα inhibitor is Etanercept, the FDA-approved dosage of 5 mg/kg at Weeks 0, 2, and 6 is preferred. In another embodiment the dose is the same as used in the Phase I TNFα inhibitor monotherapy trial (0.4 mg/kg (max 25 mg) SQ twice weekly × 26 weeks). In most preferred another embodiment only two doses are used, max 25mg/dose in combination treatment regimen including vitamin D and autoantigens.

According to certain embodiments, the TNFα inhibitor is administered prior to the first administration of the at least one antigen.

In some embodiments, the compound that reduces the immune system's ability to activate naive TCs and BCs and recall responses from activated and memory lymphocytes is a CTLA-4 compound, such as abatacept. According to particular embodiments, when the compound is abatacept doses of at least 2-20mg/kg abatacept, is administered per treatment occasion, starting within +/- 7 days around the time of first administration of the at least one antigen.

According to certain embodiments, the CTLA-4 compound is administered simultaneously with the first administration of the at least one antigen.

According to certain embodiments, administration of the at least one antigen and the compound that reduces the immune system's ability to activate naive TCs and BCs and recall responses from activated and memory lymphocytes, is repeated on day 14, 28 and 45 +/- one week as the case may be to ensure blockage of CD28 on TCs, and where day one marks the first administration of the at least one IL-10 inducing compound.

In one aspect, the invention provides a method to treat one or more symptoms associated with T1D. Symptoms associated with T1D include, but are not limited to, reduced insulin production, reduced insulin sensitivity, high blood glucose levels, destruction of insulin producing cells, and abnormal C peptide levels.

The methods of the invention may be directed towards the treatment and prevention of not only T1D but generally for autoimmune diseases and disorders. For example, subjects suffering from Grave's disease, Hashimoto's thyroiditis, hypoglyceimia, multiple sclerosis, mixed essential cryoglobulinemia, systemic lupus erthematosus, Rheumatoid Arthritis (RA), Coeliac disease, T1D, or any combination thereof. In these cases, for the disease relevant autoantigens are to be included as autoantigens in the treatment methods. In one aspect of the invention, the subjects suffer from autoimmune responses that involve T-cells or B-cells that have an antigenic specificity, or T-cell receptor (TCR) and/or B-cells that have T-cell receptor (TCR) or B-cell receptor (BCR) antigen specificity for an autoantigen.

According to certain embodiments, the individual to be treated according to the presentinvention is a mammal. According to particular embodiments, the individual to be treated according to the present invention is a human. According to certain embodiments, the individual to be treated according to the present invention is an infant. According to particular embodiments, the individual to be treated according to the present invention is an adolescent. human. According to particular embodiments, the individual to be treated according to the present invention is an adult human.

In some embodiments the human treated subject is above 4 years of age

In other embodiments the human treated subject is 8 years or above.

In other embodiments the human treated subject is 10 years or above.

In some embodiments the human treated subject is 18 years or below.

In some embodiments the human treated subject is 4-10, or 4-18, or 8-18, or 10-18 years of age.

In other embodiments the human treated subject is 18 years or above.

In some embodiments the human treated subject is 18-30 years of age.

### IL-10 inducing compounds

In some aspects, the methods, compositions and kits of the present invention use IL-10 inducing compounds.

According to certain embodiments, the at least one IL-10 inducing compound is vitamin-D, such as 1,25-Dihydroxyvitamin D.

According to certain other embodiments, the at least one IL-10 inducing compound is a vitamin-D analog, such as TX527.

According to certain other embodiments, the at least one IL-10 inducing compound include enhancement of serum vitamin D by means of UVB radiation.

According to certain other embodiments, the at least one IL-10 inducing compound is a tyrosine kinase inhibitor, such as dasatinib, bosutinib, saracatinib, imatinib, sunitinib, or combinations thereof.

According to particular embodiments, the tyrosine kinase inhibitor is dasatinib.

According to other particular embodiments, the tyrosine kinase inhibitor is bosutinib.

According to other particular embodiments, the tyrosine kinase inhibitor is saracatinib.

According to other particular embodiments, the tyrosine kinase inhibitor is imatinib.

According to other particular embodiments, the tyrosine kinase inhibitor is sunitinib.

According to other particular embodiments, the tyrosine kinase inhibitor is a combination of at least two of dasatinib, bosutinib, saracatinib, imatinib and sunitinib. For example, the tyrosine kinase inhibitor may be a combination of dasatinib and bosutinib. According to other more particular embodiments, the tyrosine kinase inhibitor is a combination of dasatinib and saracatinib. According to other more particular embodiments, the tyrosine kinase inhibitor is a combination of dasatinib and imatinib. According to other more particular embodiments, the tyrosine kinase inhibitor is a combination of dasatinib and sunitinib. According to other more particular embodiments, the tyrosine kinase inhibitor is a combination of bosutinib and saracatinib. According to other more particular embodiments, the tyrosine kinase inhibitor is a combination of bosutinib and imatinib. According to other more particular embodiments, the tyrosine kinase inhibitor is a combination of bosutinib and sunitinib. According to other more particular embodiments, the tyrosine kinase inhibitor is a combination of imatinib and sunitinib. According to other more particular embodiments, the tyrosine kinase inhibitor is a combination of dasatinib, bosutinib and saracatinib.

The Composition according to the invention may comprise more than one IL-10 inducing compound. Thus, according to certain embodiments, the Composition comprises at least two IL-10 inducing compounds. According to certain other embodiments, the Composition comprises at least three IL-10 inducing compounds. According to certain other embodiments, the Composition comprises at least four IL-10 inducing compounds.

### Cyclooxygenase inhibitors

In some aspects, the methods, compositions and kits of the present invention use one or more cyclooxygenase inhibitors.

These cyclooxygenase inhibitors may be non-steroidal anti-inflammatory drugs (NSAID). According to more particular embodiments, the NSAID is selected from the group consisting of Ibuprofen, Dexibuprofen, Naproxen, Fenoprofen, Ketoprofen, Dexketoprofen, Flurbiprofen, Oxaprozin, Loxoprofen, Indomethacin, Tolmetin, Sulindac, Etodolac, Ketorolac, Diclofenac, Aceclofenac, Nabumetone, Aspirin (acetylsalicylic acid), Diflunisal (Dolobid), Salicylic acid, Salsalate (Disalcid), Piroxicam, Meloxicam, Tenoxicam, Droxicam, Lornoxicam, Isoxicam, Mefenamic acid, Meclofenamic acid, Flufenamic acid, Tolfenamic acid, Celecoxib, Rofecoxib, Valdecoxib, Parecoxib, Lumiracoxib, Etoricoxib, and Nimesulide.

According to certain embodiments, the cyclooxygenase inhibitor is a propionic acid derivative, such as Ibuprofen, Dexibuprofen, Naproxen, Fenoprofen, Ketoprofen, Dexketoprofen, Flurbiprofen, Oxaprozin or Loxoprofen.

According to certain embodiments, the cyclooxygenase inhibitor is a Acetic acid derivative, such as Indomethacin, Tolmetin, Sulindac, Etodolac, Ketorolac, Diclofenac, Aceclofenac or Nabumetone.

According to certain embodiments, the cyclooxygenase inhibitor is a Salicylate, such as Aspirin (acetylsalicylic acid), Diflunisal (Dolobid), Salicylic acid or Salsalate.

According to certain embodiments, the cyclooxygenase inhibitor is an enolic acid (Oxicam) derivative, such as Piroxicam, Meloxicam, Tenoxicam, Droxicam, Lornoxicam or Isoxicam.

According to certain embodiments, the cyclooxygenase inhibitor is an anthranilic acid derivative, such as Mefenamic acid, Meclofenamic acid, Flufenamic acid or Tolfenamic acid.

According to certain embodiments, the cyclooxygenase inhibitor is selective COX-2 inhibitor, such as Celecoxib, Rofecoxib, Valdecoxib, Parecoxib, Lumiracoxib or Etoricoxib.

According to more particular embodiments, the cyclooxygenase inhibitor is Ibuprofen.

According to more particular embodiments, the cyclooxygenase inhibitor is Dexibuprofen.

According to more particular embodiments, the cyclooxygenase inhibitor is Naproxen.

According to more particular embodiments, the cyclooxygenase inhibitor is Fenoprofen

According to more particular embodiments, the cyclooxygenase inhibitor is Ketoprofen.

According to more particular embodiments, the cyclooxygenase inhibitor is Dexketoprofen.

According to more particular embodiments, the cyclooxygenase inhibitor is Flurbiprofen.

According to more particular embodiments, the cyclooxygenase inhibitor is Oxaprozin.

According to more particular embodiments, the cyclooxygenase inhibitor is Loxoprofen.

According to more particular embodiments, the cyclooxygenase inhibitor is Indomethacin.

Ibuprofen mainly blocks cox-2 but to some extent also cox-1. Ibuprofen has a somewhat broader effect on the immune system than a narrow IL-1 blocker and a quite pronounced anti-inflammatory effect without serious risks. Use of Ibuprofen dampens beta cell inflammation and enables the Vit D enforced DCs to induce tolerance to peptides from autoantigens presented to T-cells, thereby protecting the beta cells in the subject.

### CTLA4 compounds

In some aspects, the methods, compositions and kits of the present invention use a CTLA-4 compound, such as a cytotoxic T-lymphocyte-associated antigen 4 immunoglobulin.

According to more particular embodiments, the CTLA-4 compound is abatacept.

Abatacept (an Fc modified CTLA4 immunoglobulin) is a Tcell depleting, immunomodulating, fusion protein consisting of the extracellular portion of human CTLA4 and the heavy chain of human IgG₁. It blocks the costimulatory signal involved in activation of naive Tcells. Its ligation with CD80/86 on APCs may also interfere with and reduce CD80/86 induced IL-6, which may downregulate inflammatory cytokines such as IL-1beta, IFNgamma, and IL-17. Further Abatacept ligation with CD80/86 may induce indoleamine dioxygenase (IDO) in APCs, which may in turn may induce anergy in Tcells, as well as downregulate paracrine activation of naïve Tcells by activated Tcells. CD80/86 expressed on B cells may yet be a further path where abatacept may carry out an immunomodulating function.

### TNFalpha inhibitors

In some aspects, the methods, compositions and kits of the present invention use a TNF alpha inhibitor, such as Adalimumab, Certolizumab, Etanercept, Golimumab or Infliximab. According to more particular embodiments, the TNF alpha inhibitor is Adalimumab. According to other more particular embodiments, the TNF alpha inhibitor is Certolizumab. According to other more particular embodiments, the TNF alpha inhibitor is Etanercept. According to other more particular embodiments, the TNF alpha inhibitor is Golimumab. According to other more particular embodiments, the TNF alpha inhibitor is Infliximab.

The invention assumes the understanding of conventional molecular biology methods that include techniques for manipulating polynucleotides that are well known to the person of ordinary skill in the art of molecular biology. Examples of such well known techniques can be found in Molecular Cloning: A Laboratory Manual 2nd Edition, Sambrook et al., Cold Spring Harbor, N.Y. (1989). Examples of conventional molecular biology techniques include, but are not limited to, in vitro ligation, restriction endonuclease digestion, PCR, cellular transformation, hybridization, electrophoresis, DNA sequencing, and the like.

The invention also assumes the understanding of conventional immunobiological methods that are well known to the person of ordinary skill in the art of immunology. Basic information and methods can be found in Current Protocols in Immunology, editors Bierer et al., 4 volumes, John Wiley & Sons, Inc., which includes teachings regarding: Care and Handling of Laboratory Animals, Induction of Immune Responses, In Vitro Assays for Lymphocyte Function, In Vivo Assays for Lymphocyte Function, Immunofluorescence and Cell Sorting, Cytokines and Their Cellular Receptors, Immunologic Studies in Humans, Isolation and Analysis of Proteins, Peptides, Molecular Biology, Biochemistry of Cell Activation, Complement, Innate Immunity, Animal Models for Autoimmune and Inflammatory Disease (which includes chapters on the NOD mouse model, the SLE mouse model (for lupus), and induction of autoimmune disease by depletion of regulatory T cells), Antigen Processing and Presentation, Engineering Immune Molecules and Receptors, Ligand-Receptor Interactions in the Immune System, Microscopy, and Abbreviations and Terminology for common immune system genes and proteins, including the CD system for Leukocyte Surface Molecules.

### Experimental

The examples provided below are intended to illustrate some aspects and embodiments of the invention. They should not be construed as limiting the scope of the invention, which is that of the appended claims.

### Example 1

### Methods

### Procedure

A group of T1D patients (n=6) participating in a pilot trial (NCT02352974), received a primary injection of 4 µg of GAD-alum (Diamyd Medical, Stockholm) into an inguinal lymph gland, followed by two booster injections of 4 µg each with one month interval. In parallel they got Vitamin D 2000 U/d. Another control group of patients (n=6) who received two subcutaneous injections of GAD-alum, 20 µg each, one month apart, who also in parallel they got Vitamin D 2000 U/d, were selected under blind conditions, before code break, to match age and sex, and according to disponibility of samples among participants in a double blind placebo-controlled study described elsewhere (NCT01785108) **(****Fig 1****).**

### Laboratory Tests

Laboratory analyses were performed at Linköping University, Sweden. Blood and serum samples were collected at baseline and after 30, 60, 90 and 180 days in LN group, and after 15, 45, 90 and 180 days in SC group. Samples were drawn during the morning hours and PBMCs were isolated within 24 h using Leucosep (Greiner Bio One) according to the manufacturer's instructions.

### Serum antibodies and IgG subclasses

Serum GAD autoantibodies (GADA) and IA-2A were estimated in duplicate by means of a radiobinding assay, using ³⁵S-labeled recombinant human GAD₆₅ as previously described. Sepharose protein A was used to separate free from antibody-bound labelled GAD₆₅. A diabetes autoantibody standardization program (IASP) in which the laboratory participated has shown that GADA assay has a sensitivity of 70% and specificity of 100% and for IA-2A was 99% sensitivity and 100% specificity.

Total serum IgE was quantified using the ImmunoCap100€ system (Phadia AB, Uppsala, Sweden). The measuring range for the assay was 2-50 000 kU/L, and calibrators were run in duplicates to obtain a full calibration curve. Levels of total IgE > 85 kU/L were regarded as positive.

### Lymphocyte proliferation assay

For proliferation assays, PBMC were re-suspended at 10⁶ cells/ml in AIM-V medium, and incubated in triplicates (2×10⁵ cells/well) in round-bottom 96-well plates in presence of 5 µg/ml rhGAD₆₅ (Diamyd Medical, Stockholm, Sweden), CD3/C28 beads (Gibco, Life Technologies AS, Oslo, Norway) as positive control, and in medium alone. After 3 days, cells were pulsed for 18 hours with 0.2 µCi of [³H] thymidine/well (Perkin Elmer), and thereafter harvested. Proliferation was recorded using a 1450 Wallac MicroBeta counter (Perkin Elmer, Shelton, CT, USA), and expressed as stimulation index (SI), calculated as the mean of triplicates for each culture condition divided by the mean of triplicates with medium alone.

### Cytokine secretion assay

For cytokine quantification and flow cytometry, one million PBMC diluted in 1 ml AIM-V medium supplemented with 20 µM β-mercaptoethanol (Sigma-Aldrich, Saint Louis, MO, USA) were cultured for 7 days at 37°C in 5% CO₂ in the presence of 5 µg/ml rh-GAD₆₅. Additional, wells with medium alone and CD3/CD28 beads were used as negative and positive controls respectively for each sample. After 7 days, PBMC were separated from supernatants, and supernatants were preserved at -70 until used for multiplex fluorochrome analysis, and cells were directly used for flow cytometry.

The cytokines interleukin (IL)-2, IL-5, IL-10, IL-13, IL-17, tumour necrosis factor (TNF)-α and interferon (IFN)-y were measured in cell culture supernatants using Bio-Plex Pro Cytokine Panel (Bio-Rad, Hercules, CA, USA) according to the manufacturer's instructions. Data was collected using the Luminex 200 ^{™} (Luminex xMAP^{™} Corporation, Austin, TX USA). The lowest limit of detection was 1.38 pg/ml for IL-2, 1.27 pg/ml for IL-5, 0.14 pg/ml for IL-10, 0.43 pg/ml for IL-13, 1.54 pg/ml for IL-17, 2.1 pg/ml for IFN-y and 3.71 pg/ml for TNF-α.. The specific antigen-induced cytokine secretion level was calculated by subtracting the spontaneous secretion (i.e. secretion from PBMC cultured in medium alone) from the one following stimulation with GAD₆₅.

### Flow cytometry

For flow cytometry analysis, PBMC were incubated in AIM-V medium with β-mercaptoethanol at 37 °C, 5% CO2 for 7 days, with or without 5 µg/ml recombinant GAD₆₅. After incubation, cells were washed in PBS containing 0.1% BSA, and subsequently stained with Alexa-700-conjugated anti-CD3 (clone UCHT1, BD Biosciences), Pacific Blue-conjugated anti-CD4 (clone RPA-T4, BD Biosciences), allophycocyanin (APC)-H7-conjugated anti-CD8 (clone SK1, BD Biosciences), PerCP-Cy5.5-conjugated anti-CD45RA (clone HI100, BD Biosciences), phycoerythrin (PE)-conjugated anti-CCR7 (clone G043H7, Biolegend), FITC-conjugated anti-CD127 ( clone eBioRDR5, eBioscience ) and PE-Cy7-conjugated anti-CD25 (clone BC96, eBioscience). After surface staining, cells were fixed and permeabilized using FOXP3 staining buffer set (eBioscience), according to the manufacturer's instructions. Cells were then stained with APC-conjugated anti-FOXP3 (clone PCH101, eBiosciences) and acquired on a FACS Aria III (Becton Dickinson) running FACS Diva v8 software (Becton Dickinson). Data were analyzed using Kaluza v1.3 (Beckman Coulter).

### Statistical analysis

Data distribution was tested using Komolgorov-Smirnov test. Variables that followed a normal distribution were presented as mean, and differences within groups were calculated by Paired samples test's. Differences between groups were calculated using T-student test. For non-normally distributed variables, non-parametric test were applied (Wilcoxon test related samples and Mann-Whitney test). Differences between categorical variables were calculated by Chi-square test (χ² - test). A probability level of <0.05 was considered statistically significant. Calculations were performed using IBM SPSS Statistics version 23 (IBM SPSS, Armonk, NY, USA) and graphical illustrations were made in GraphPad Prism 5 for Windows (GraphPad Software, La Jolla, CA, USA).

### Results

Patients were stratified into those who received lymph-node (LN) or subcutaneous (SC) GAD-alum injections. Gender distribution was the same in both groups, while mean age was higher in LN patients (22 years) than in the SC group (14 years) (p<0.001). At baseline, both groups had similar baseline mean C-peptide (fasting, max. stimulated and AUC). Pre-treatment HbA1c values were higher in LN patients, who had lower insulin doses (p>0.05). GADA and IA-2A autoantibody levels did not differ between the groups (Table 1).

Follow up of the patients showed that fasting and stimulated c-peptide (AUC) remained stable at 180 days in the LN group, while glycated hemoglobin levels and insulin intake decreased. Patients in SC group had a greater loss of stimulated c-peptide, as well as higher glycated hemoglobin levels and increased insulin intake (Table 1).

### GADA titers and GADA subclasses analysis

GADA levels were enhanced after the second injection of GAD-alum both given SC and LN (Figure 2a). However, LN administration of low GAD-alum doses induced GADA levels 29 times higher than SC injection of higher doses.

We looked next to the GADA IgG 1-4 subclass distribution, calculating the frequencies of each subclass with respect to the combined sum of all the subclasses in each sample (i.e total IgG). Baseline GADA subclass distribution was similar in the two groups, being IgG₁ the most frequent, and followed by IgG₃>IgG₂≈IgG₄ (Figure 2b).The proportion of IgG₁ decreased from baseline to 90 days both in the LN and SC groups, while the proportion of the other subclasses increased. Intriguingly, while GADA subclasses distribution in the SC group at 180 days was similar to that observed at baseline, the proportion of IgG₁ in the LN group was further reduced, with a dramatically increase of IgG₂, IgG₃ and IgG₄ (Figure 2c).

To disregard a possible allergy-associated effect in response of GAD-alum, total IgE was measured at baseline and at 180 days. Results showed that baseline IgE levels were similar in patients receiving intralymphatic and subcutaneous injections, and levels were not affected by the treatment and remained unchanged after 180 days (data no shown).

### Cytokine secretion and relative contribution

We next analyzed the cytokine secretion in PBMC supernatants collected after 7 days culture. Baseline cytokine secretion was similar in the two groups.

GAD₆₅-induced secretion of IL-5, IL-13 IFN-y and IL-17 was increased at 90 days, both after the second GAD-alum SC and LN doses, together with IL-2 in the SC group. The third GAD-alum injection into the LN resulted in a predominant secretion of IL-13 and low levels of IFNy at 180 days months. Meanwhile, IFNy was the most secreted cytokine in the SC group at the same time point (Figure 3a).

We further assessed the relative contribution of each cytokine to the total GAD₆₅-induced cytokine secretion. In the LN group, a broad cytokine profile was observed at 90 days, following the second injection of GAD-alum, while cytokine secretion at 180 days, after the third injection, was dominated by the Th2-associated cytokine IL-13. In the SC group, cytokine profile was also characterized by a broad cytokine secretion at 90 days with a predominant secretion of Th2 cytokines, but cytokine distribution shifted into a dominant Th-1-like response at 180 days (Figure 3b).

### In vitro stimulation with GAD₆₅

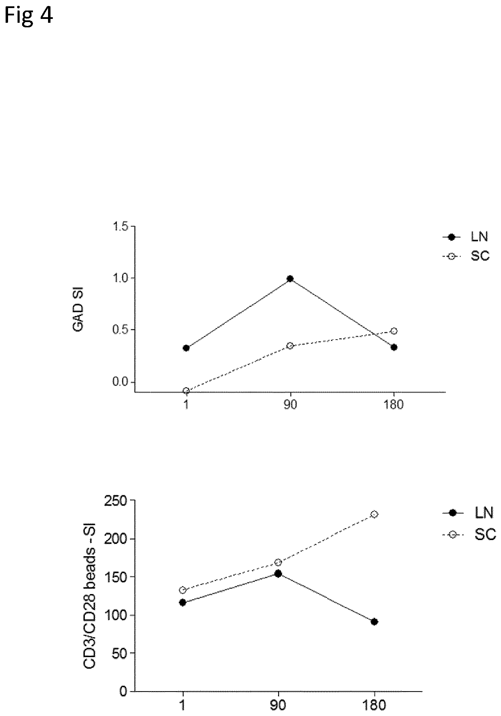

GAD₆₅₋induced proliferation was increased by the second injection of GAD-alum both into the LN and SC. The third injection to the LN group resulted in a reduction of proliferation at 180 days, while remained stable in the SC group. Proliferation induced by stimulation with CD3/CD28 beads showed the same distribution as the induced by GAD₆₅ (Figure 4).

### T cell immunophenotype

We monitored the differentiation state and GAD₆₅₋induced activation of T cells. A representative illustration of the gate strategy followed for the analysis of CD8, CD4 and regulatory T cells is shown (figure 5).

GAD₆₅ stimulation induced activated CD25⁺CD127⁺ T cells in both groups after the second injection of GAD-alum. In the LN, higher frequency of activated CD4 T cells was detected in 3 patients, while activation of CD8 T cells was moderate or not detectable. In the SC group in contrast, the proportion of activated CD8 T cells was more predominant, with weaker expression of GADss-activation of CD4 cells (Figure 6 a-b)

The analysis of CD4⁺FOXP3⁺CD25^{hi}CD127^{low/-} Tregs showed that resting Treg did not vary through the study, but antigen recall induced an increase in cells with regulatory phenotype at 180 days in both groups (Figure 6 c-d). Further analysis with the addition of CD45RA revealed an increment in non-suppressive FOXP3^{lo} CD45RA T cells in both groups (Figure 6 e-f).

CD4 and CD8 T cells were further classified according to the expression of CD45RA and CCR7 as näive (T_{N}, CD45RA⁺CCR7⁺), central memory (T_{CM}, CD45RA⁻CCR7⁺), effector memory (T_{EM}, CD45RA⁻CCR7₋) and terminally differentiated effector memory (T_{EMRA}, CD45RA⁺CCR7⁻) cells (Figure 7). Both groups, LN and SC, showed a progressive reduction in the proportion of näive CD4 and CD8 T cells after 90 days, while the frequency of memory and effector cells increased in GAD₆₅ stimulated PBMC (Figure 7 a-h).

**Table 1**

| Baseline | | | | | | | | | | | | | |
|---|---|---|---|---|---|---|---|---|---|---|---|---|---|
| | Id | Sex | Age | Fasting C-peptide | Max. Stim | AUC | Insulin intake | HbA1c | GADA (U/ml) | IgG₁ (%) | IgG₂ (%) | IgG₃ (%) | IgG₄ (%) |
| L N | 1 | M | 23 | 0.12 | 0.61 | 0.43 | 0.29 | 66 | 929 | 70 | 29 | 0.4 | 0.9 |
| | 2 | M | 22 | 0.26 | 1.13 | 0.85 | 0.25 | 58 | 111 | 25 | 55 | 5.8 | 14.5 |
| | 3 | M | 21 | 0.26 | 0.62 | 0.42 | 0.29 | 103 | 968 | 64 | 9 | 11.5 | 14.7 |
| | 4 | F | 21 | 0.25 | 1.26 | 0.74 | 0.45 | 68 | 2955 | 91 | 0.3 | 8.7 | 0.3 |
| | 5 | M | 23 | 0.16 | 0.73 | 0.38 | 0.55 | 78 | 14100 | 92 | 0.2 | 5.6 | 2.7 |
| | 6 | F | 21 | 0.17 | 0.58 | 0.36 | 0.46 | 52 | 27450 | 43 | 17 | 17.9 | 21 |
| S C | 1 | M | 13 | 0.18 | 0.99 | 0.69 | 0.55 | 47 | 114 | 39 | 8 | 16 | 37 |
| | 2 | F | 17 | 0.59 | 0.9 | 0.8 | 1.36 | 68 | 786 | 64 | 6 | 19 | 10.2 |
| | 3 | M | 17 | 0.24 | 0.77 | 0.6 | 0.6 | 65 | 667 | 87 | 1.4 | 10.4 | 1.4 |
| | 4 | F | 11 | 0.15 | 0.56 | 0.46 | 0.38 | 50 | 70 | 10 | 59 | 28 | 3 |
| | 5 | M | 15 | 0.48 | 1.12 | 0.97 | 0.42 | 33 | 472 | 87 | 1.7 | 9 | 2.6 |
| | 6 | M | 11 | 0.18 | 0.61 | 0.44 | 0.48 | 41 | 1215 | 85 | 4.7 | 3.6 | 7 |

| IA-2 (U/ml) | IL-13 pg/ml | II-5 pg/ml | IL-10 pg/ml | IL-2 pg/ml | IFN pg/ml | TNF pg/ml | IL-17 pg/ml | ΔFast C-peptide | | ΔMax. Stim | ΔAUC | ΔInsulin intake | ΔHbA c |
|---|---|---|---|---|---|---|---|---|---|---|---|---|---|
| 704 | n.d | 8.6 | 0.95 | n.d | n.d | n.d | n.d | 108.3 | | 13.1 | 30.2 | -48.2 | -31.8 |
| 266 | n.d | 0.6 | n.d | n.d | n.d | n.d | n.d | -23 | | 7.9 | -1.1 | -4 | -32.7 |
| 497 | n.d | n.d | n.d | n.d | n.d | n.d | n.d | -26.9 | | 3.2 | 21.4 | -41.3 | -55.3 |
| 13.6 | n.d | n.d | n.d | 0.61 | n.d | n.d | n.d | -4 | | -11.1 | 6.7 | -64.4 | -47 |
| 2.7 | n.d | n.d | n.d | n.d | n.d | n.d | n.d | -25 | | -31.5 | -13.1 | -27.2 | -44.8 |
| 2.7 | n.d | 0.6 | n.d | n.d | n.d | n.d | n.d | -47 | | -31 | -27.7 | 17.3 | -15.3 |
| 23 300 | n.d | n.d. | 0.25 | n.d | n.d | n.d | n.d | 77.7 | | -22.2 | -5.7 | -54.5 | 2.1 |
| n.d | n.d | 0.3 | n.d | n.d | 1976 | 37 | n.d | -18.6 | | 7.7 | 6.2 | 9.5 | -19.1 |
| 2 880 | 969 | 80.5 | 3.74 | n.d | n.d | n.d | 10.2 | -20.8 | | -15.5 | -11.6 | -46.6 | -55.3 |
| 619 | n.d | n.d | 1.35 | n.d | n.d | 0.78 | 1.3 | 153.3 | | -28.5 | -19.5 | 65.7 | 48 |
| n.d | n.d | n.d | n.d | n.d | n.d | n.d. | n.d | -41.6 | | -32.1 | -35.0 | 16.6 | 36.3 |
| 4.7 | n.d | n.d | n.d | 2.36 | n.d | n.d | n.d | -94.4 | | -60.6 | -65.9 | 41.6 | 39.0 |

| | | | | | | | | | | | | | |
|---|---|---|---|---|---|---|---|---|---|---|---|---|---|
| Baseline C-peptide, insulin intake and HbA1c of the patients who received GAD-alum injections into the lymph-nodes (LN, n=6) or subcutaneously (SC, n=6). Pre-treatment GADA and IA-2 titers (U/ml), GADA IgG subclass relative distribution (%), and GAD₆₅₋induced cytokine secretion levels (pg/ml) from each patient. M: male, F: female, n.d: non detectable levels. | | | | | | | | | | | | | |

Percentage of change (%) of C-peptide, insulin intake and HbA1c from baseline to 180 days. Data are represented as mean values, and differences between LN and SC groups were calculated by T-student test, and χ² for categorical variables (sex). Differences were considered significant for p<0.05.

### Example 2

The following example discloses studies that may be performed in order to establish the safety and efficacy of various aspects of the present invention. The beta cell autoantigen used in the examples is glutamic acid decarboxylase (GAD), and may be replaced with any other beta cell autoantigen as described herein.

### Pilot Trial to preserve residual insulin secretion in adults with recent-onset Type 1 diabetes by giving GAD-antigen (Diamyd^{®}) therapy into lymph nodes. (DIAGNODE)

### 1.1 Background and rationale

The incidence of Type 1 diabetes (T1D) in children is next to Finland highest in Sweden in the world, and is increasing rapidly. T1D is by far the most commonchronic, serious, life-threatening disease among children and adolescents in our country, and the incidence of Type 1 diabetes is high also in young adults. The disease tends to become an extremely serious global problem. The disease is characterized by lack of insulin. Even though several patients at diagnosis have rather impressive residual beta cell function (1) the deficiency becomes soon very pronounced and finally complete (2,3). Residual insulin secretion is of crucial importance. In rare cases the beta cell function improves so much shortly after diagnosis that glucose metabolism normalizes and no insulin is required for some time, that is the patient goes into so called complete remission (4). As long as the patient is in a complete remission there is no need of active treatment, more than perhaps some recommendation of sound life style regarding physical exercise and diet. There are no symptoms, no acute complications and if somebody stayed in complete remission it is unlikely that such an individual would ever develop late complications. Slight abnormality of glucose or lipid metabolism might increase the risk of macrovascular complications in the same way as for individuals with chemical diabetes or impaired glucose tolerance. Complete remission is rare, but partial remission it is not (4). During this period the patient usually has near normal blood glucose values, not even mild hypoglycemia and no episodes of ketoacidosis. The quality of life is very good as the patient feels well, children grow normally, few restrictions are needed, if any with regard to food, the patients can exercise with great variation without getting hypoglycemia, and experiences very good home blood glucose tests. Only some residual insulin secretion is enough to diminish the risk of ketoacidosis (5). Furthermore, it has been shown in the DCCT trial that even quite modest residual insulin secretion, a response to a beta cell stimulation with serum Cpeptide >0.20pmol/ml, plays an important role for prevention of complications (6). This effect may be due to the fact that residual insulin secretion should reasonably make it easier to reach good blood glucose balance, but it is also possible that Cpeptide per se has a physiological function. It has in fact been reported that Cpeptide influences vascular permeability, decreases leakage in retinal vessel, and not least has a positive effect on nerve function (7) although the effect of C-peptide per se still is under debate.

### 1.1.1 Factors influencing the natural course

At diagnosis of T1D it has been claimed that 80-90% of the beta cells in pancreas have been destroyed. However, the proof for this is scarce, and it may well be that the main problem is deterioration of function. Furthermore there is great difference between patients, as some have quite good residual insulin secretion and others have not. Shortly after diagnosis, especially when an active insulin treatment is given, there is an increase of C-peptide production, and at the same time an improvement of insulin sensitivity. Good metabolic control seems to improve the milieu and metabolism for the beta cells and the beta cell function is preserved, which in turn contributes to better metabolic control, and vice versa. The intensity of the autoimmune process plays a role, and it seems evident that children have a more aggressive immune process than adults with Type 1 diabetes, but it is still difficult to predict the course. Some studies have suggested that high concentrations of autoantibodies are followed by a more rapid loss of insulin secretion, while others have not found such a relationship, or even the opposite. No special signs of cell-mediated immunity have so far been proven to predict beta cell loss but our own studies have shown that disease process is related to a T-helper-1 (Th-1) deviation of the immune system with increases of certain cytokines such as IFNg and decrease of IL-10, IL-13.

### The effect of insulin treatment on beta cell function.

Active insulin treatment during the first period of the disease prolonged the partial remission long time ago, and this finding could be confirmed and validated by improved residual insulin secretion (2). Intensified treatment seems to improve residual beta cell function at least for some time (8), but it may also have long-term positive effects (9). Active treatment has been shown not only to prevent or postpone diabetes in experimental animals, but studies have indicated that such treatment could prevent diabetes in high risk individuals (10). However, when tried at a larger scale in the Diabetes Prevention Trial, parenteral insulin treatment did not prevent diabetes (11). Oral treatment with insulin might have an effect (12) and therefore further studies are needed.

### 1.1.2 Interventions

In the 1970ies it became clear that T1D is an autoimmune disease and therefore immune interventions were tried. We performed the first immune intervention studies in the world on diabetic children when we already 30 years ago used plasmapheresis in newly-diagnosed children and adolescents with some positive effects (13). As a side effect of that treatment a new protein with the weight 64kD was found in plasma (14), which later showed to be Glutamic Acid Decarboxylase (GAD). The breakthrough, taken as a proof for the concept of immune intervention, was cyclosporin, which doubtless slowed down the autoimmune destructive process and gave improved residual insulin secretion, while other trials with immune suppression had minimal effect, especially so in children (15, 16,17), or showed too serious adverse events or risks (18, 19). In an effort to modulate the immune system we used photopheresis. Although clear effects on the immune system were demonstrated in a double blind placebo-controlled trial (20), the clinical effect was minimal and almost no improvement of residual beta cell function could be seen (21). Thus, with no successful immune intervention, our interest was directed to protective agents such as Nicotinamide and Diazoxide, with no or transient effect (22, 23, 24).

With increasing knowledge of the immune process leading to beta cell destruction, it has become possible to direct more precisely the immune intervention to target the important T-cells. Promising studies using anti-CD3antibodies in an attempt to block the destructive immune process have been performed. Results from both North- American and French trials with anti-CD3 have shown that it is possible to block the destructive autoimmune process and thereby at least postpone the decline of the beta cell function (25,26). The decline of residual insulin secretion was significantly slowed down, but unfortunately it looks as if the decline was just delayed a year, and thereafter the declining Cpeptide curve went parallel to the declining curve in the placebo group. Furthermore, a majority of the patients experience some Cytokine Release Syndrome (CRS), which may be quite serious, and in addition a number of side effects were seen in most of the patients. We have participated in one of two recent Phase III trials (Protegé trial), which failed to reach the primary endpoint, although the arm with the most intense treatment indeed showed some preservation of residual insulin secretion and lower insulin requirement to reach good HbA1c (27; Sherry, Hagopian, Ludvigsson et al Lancet 2011). New studies are needed but it is difficult to believe that this type of treatment alone will be the accepted solution for general clinical use. Even less likely is such a treatment accepted as a preventive treatment in otherwise healthy individuals of whom many never would develop diabetes.

### 1.1.3 Immune therapy with auto-antigens

In the treatment of allergic diseases, immunotherapy with small amount of disease specific antigen has been efficiently used during many years.The mechanism for this treatment remains unclear, although immune modulation of the immune responses and induction of regulatory cells have been suggested. In autoimmune diseases no such treatment has been successful, but should be tried (28). Experiments in diabetes prone animals have shown that treatment with a heat shock protein could delay or postpone development of diabetes. The use of Diapep277 peptide in a study in adults showed significant preservation of insulin secretion without almost any adverse events (29). Later trials in children and adolescents with T1D (30), however, have shown no effect. Studies with Diapep277 treatment in so called LADA (Latent Autoimmune Diabetes in the Adult) are ongoing, and preliminary results (report at IDF, Dubai Dec 2011 and at ADA June 2012) suggests that treatment with Diapep277 may preserve beta cell function in adults with mild Type 1 diabetes. However, the results are a bit unclear, as there was a weak C-peptide preservation only seen after Glucagon stimulation, but no effect at all after Mixed Meal Tolerance Test, and there was no differences whatsoever between the actively treated group and placebo in immune markers. Active treatment with insulin has been shown not only to prevent or postpone diabetes in experimental animals but preliminary open studies indicated that such treatment could prevent diabetes in high risk individuals (10). Insulin, clearly a beta cell specific auto-antigen, has been parentally administrated (DPT) to prevent diabetes in high risk individuals with no effect, while oral insulin administration with the same purpose may have a slight effect (12).

### 1.1.4 Previous clinical studies with GAD-Alum

### 1.1.4.1 GAD-vaccination

GAD (Glutamic Acid Decarboxylase), can be regarded as an auto-antigen, as it is produced in the islets with increased release as response to beta cell stimulation. This protein has been shown to deeply influence the autoimmune immune process (31,32,33,34). Several studies have shown that indeed GAD can prevent diabetes in experimental animals (35-42). The similarity of GAD with viral proteins may be important for the therapeutic action. The observed effect, even after the start of the immune process, suggests that it might be possible to expect the same effect in humans after the start of the immune process. In a phase II study in LADA patients the administration of one low dose, Diamyd 20 µg, led to improved beta cell function for up to 2 years compared to the placebo treated group, with no side effects. Also other doses were tried: 4 µg showed no effect, 100 µg showed a similar effect as 20 µg, while 500 µg showed no effect. None of the doses showed any adverse events, still so after several years follow-up (43). Association with change in the ratio of CD4+CD25+/ CD4- CD25- cells was found, indicating a mechanism for the effect. With this promising background we started a Phase II study in Type 1 diabetic patients 10-18 years with recent onset. Based on the idea that the treatment earlier had effect in slowly progressive LADA patients we included patients with up to 18 months duration of T1D diabetes at intervention. The patients were randomized to either 20 µg GAD-alum (Diamyd) sc at Day 1 and 30, or placebo. The effect still after 30 months was remarkable, and clearly both statistically and clinically significant (44), with about half of the Cpeptide decline in the GAD treated group compared with the placebo group. Patients with a diabetes duration < 3 months had a remarkably good effect with no or minimal decline of beta cell function during the follow-up of the first 15 months. Almost all effect was seen in patients with < 6 months duration at vaccination. Even more, in contrast to other intervention treatments, this effect was gained with no adverse events at all, making the treatment very encouraging! Still after 48 months the patients treated with < 6 months duration hade significantly preserved C-peptide and still no adverse events (45). So far GAD-treatment looked very promising. Two Phase III trials were performed, one European with Johnny Ludvigsson (JL) as PI, and one in USA with Jerry Palmer as PI and JL as coinvestigator. In the European trial 334 patients were recruited into three arms, one arm with GAD-alum (Diamyd) 20 µg at Day 1,30, 90 and 270 ,another arm with GADalum 20 µg at Day 1 and 30, and placebo at Day 90 and 270 and a third arm with Placebo at Day 1,30,90 and 270. Primary endpoint, serum C-peptide AUC after a Mixed Meal Tolerance Test (MMTT) at 15 months was not met! (C-peptide AUC p=0.1; Fasting C-peptide p=0.07) (46). This prompted the company (Diamyd Medical + Johnson&Johnson) to close the phase III trials early. However, the Phase III trial did show several positive effects. Thus statistically significant efficacy was seen in several pre-specified subgroups. Furthermore, 45 Swedish patients had passed the 30 month's visit when the study was stopped, and those 15 patients who had received two doses of GAD-alum (Diamyd) 20 µg showed a significant preservation of C-peptide after 30 months compared with placebo! This is especially remarkable as the Swedish patients were the ones without efficacy after 15 months, while efficacy was found after 15 months in the non-Nordic countries.

### 1.1.4.2 Possible Reasons to the different results Phase II and Phase III

In Phase III randomization, patients receiving active drug were more often in the 10- 11 year age group than in the 16-18 year age group whereas placebo was more frequent than active drug in the higher age group. This may have influenced the result. The Phase II patients were treated in March-April and those patients in Phase III who were treated in March-April had also significant effect of GAD-treatment. In the Phase II trial no vaccinations were accepted, but in Phase III Influenzavaccination was allowed. Unfortunately an epidemic of H1N1-flu lead to that almost all patients were vaccinated, many of them in connection with the GAD-vaccinations.

In Sweden and Finland the vaccine contained squalen, suspected to influence the immune system towards auto-immunity, and in these two countries there was no efficacy of GAD-treatment, while the efficacy was significant in other European countries. Patients in Sweden who did not get the influenza vaccination close to the GAD-treatment, had better efficacy of GAD-treatment (46).

### 1.1.4.3 Ongoing DIABGAD-1 trial

Since Jan 2013 the Phase II DIABGAD-1 trial is ongoing in Sweden. This is a trial in 60 patients, 10-18 years old, with recent-onset Type 1 diabetes, who are treated in a double-blind placebo-controlled randomized study with GAD-alum 20 µg resp 40 µg given twice with 30 days interval, in combination with Vitamin D, 2000 units daily for 450 days, and Ibuprofen 400 mg daily for 90 days. Recruitment is fulfilled and now closed. 60 patients are randomized and another 4 patients are screened, waiting for screening results.. There are so far no Serious Adverse Events judged as related to study medication, and very slight adverse events, not related to the treatment except for mild transient reactions on the site of injection of GAD-alum. An interim analyses is planned already after 6 months follow-up of all patients while more extended analyses will be performed after 15 and 30 months.

### 1.1.5. Intra lymph-node immunotherapy

Antigen therapy aims at presenting the antigen to the T-cells in the lymph nodes to get a new balance of the immune system and tolerance against the antigen. In the treatment of autoimmune diseases so far antigen has been given either orally, intranasally or subcutaneously, in order to present the antigen to antigen presenting/dendritic cells which in turn are expected to present the antigen to the Tcells of the immune system. However, animal studies have shown that intralymphatic injections induce a strong and relevant T-cell response (47,48) and in the allergy field clinical studies have shown that presentation of the antigen/allergen directly into the lymph nodes seems to be more effective than traditional administration (49). Lower doses of the allergen can be used, the number of treatments can be radically reduced, and there have been no treatment-related adverse events. Inguinal lymphnodes are readily accessible in patients and the pain associated with the injection is rated as below that of venous puncture (50). With this background it is our aim to study whether the same approach can be used in patients with the autoimmune form of Type 1 diabetes. For ethical reasons we will do the first pilot trial in adults.

### 1.2 Hypothesis

The encouraging results of the Phase II GAD trial and the partly positive results of the Phase III European Trial, support the concept that administration of GAD-alum (Diamyd) may decrease the autoimmune process and contribute to preservation of residual insulin secretion III As previous studies have indicated that the dose should be somewhere between 20 and 100 µg Diamyd, a lower dose of 3 µg given three times should be adequate when administrated directly into lymph nodes. 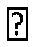 Injection of GAD-alum (Diamyd) directly into lymph nodes will give no serious adverse events, have desired immunological effects and will (shown in future studies) improve efficacy

### 2. Risk-Benefit Analyses

The incidence of Type 1 diabetes is next to Finland higher in Sweden than in any other country of the world. The incidence is continuously increasing for decades. The disease cannot be cured and cannot be prevented. In spite of a very heavy, intensive, expensive treatment many patients get life-threatening serious both acute and late complications, and the mortality is much increased. At diagnosis many patients have some slight residual insulin secretion. As long as this is the case it is much easier to keep blood glucose stable, the incidence of hypoglycaemia decreases as well as the risk of ketoacidosis. Quality of life for the patient as well as for parents of children with diabetes is better as long as there is some residual insulin secretion.

Type 1 diabetes in adults differ from the disease in children and adolescents as the disease process often is milder, the decline of residual insulin secretion slower, and it is easier to control blood glucose. However, there are still great similarities, similar treatment and complications, and preservation of beta cell function is also very important in adults.

It is evident that there is a great benefit of preservation of residual insulin secretion, and therefore therapies aiming at preservation of this function justifiy treatments that are quite heavy, even dangerous and expensive. Thus it has been regarded as justified to treat Type 1 diabetes at onset with drugs like monoclonal antibodies against the CD3-receptor, which causes adverse events in principally all patients, some even quite serious adverse events and risks. Even pure cytostatics have been used.

In our proposed study we use GAD-alum (Diamyd) 4µg × 3, a treatment which has been used in much larger doses given to children and adults with almost no adverse events seen during follow-up of thousands of patient years. In our study we plan to use a very low dose, which means that the general risk can be expected to be even lower, but the administration will be directly into a lymph node which might give local reactions. The effect on the immune system may become more pronounced but should not lead to any adverse effects. Previous studies in allergy (where one of the co-investigators, Helene Zachrisson has given the intra lymph node injections of alum-formulated allergens) have not shown any adverse effect neither generally nor locally). For safety reasons, as this is the first pilot trial ever with this type of autoantigen treatment into lymphnodes, we try first in adults who can give their free informed consent. Even though Type 1 diabetes in adults is somewhat milder than in younger patients, it is of great value to preserve beta cell function, and therefore the proposed treatment may be of great therapeutical value also for adult patients.

When summarizing the pros and cons, there is a clear possibility of therapeutic benefit of great importance, both for the participating patients, for patients in future studies, whereas the risk is very small. If these studies contribute to the development of a good treatment, this will be of enormous value for a great number of patients.

### 3. Aim of present study

Our aim of the present pilot study is to get information on whether GAD-alum can be given into lymph nodes without treatment related serious adverse events, to allow future Phase Il-studies with the same technique to improve efficacy in preserving residual insulin secretion in Type 1 diabetes. Thus we want to see whether this treatment give any adverse events, and how treatment regimens influence the immune system, cause the desired Th-2 deviation, increase of T-regulatory cells, and hopefully indications of preservation of residual beta cell function. Based on the short-term results of this pilot study (6 month follow-up) we then may want to design a larger Phase II trial, to include younger patients and to get more robust information. The main long-term goal is then to find a treatment at onset of Type 1 diabetes in young patients which is tolerable for the patients, safe, and which can preserve residual insulin secretion and give the patients a better quality of life, with less acute complications and in the long run less risk of late complications.

### 4. Objectives

### Objectives:

To evaluate the safety of giving Diamyd directly into lymph glands and to evaluate the immune response (51-54) and effect on preservation of endogenous insulin secretion, measured at baseline and after 6, 15 and 30 months.

### 5. Population

Adult patients with recent onset of Type 1 diabetes at Linköping university hospital are given information about the study and they are asked to participate in the trial.

### 5.1 Inclusion criteria

1. Informed consent given by patients and guardians/parents
2. Type 1 diabetes according to the ADA classification with < 6 months diabetes duration
3. Age 18.00-29.99 years at diagnosis of Type 1 diabetes
4. Fasting C-peptide ≥0.12 nmol/ml
5. Pos GADA but < 50 000 random units
6. Females must agree to avoid pregnancy and have a negative urine pregnancy test
7. Patients must agree to using adequate contraception, until 1 year after the last administration of GAD-Alum/Placebo

### 5.2 Exclusion criteria

1. Previous or current treatment with immunosuppressant therapy (although topical or inhaled steroids are accepted)
2. Continuous treatment with any inflammatory drug (sporadic treatment e.g. because of headache or in connection with fever a few days will be accepted)
3. Treatment with any oral or injected anti-diabetic medications other than insulin
4. A history of anaemia or significantly abnormal haematology results at screening
5. A history of epilepsy, head trauma or cerebro-vascular accident, or clinical features of continuous motor unit activity in proximal muscles
6. Clinically significant history of acute reaction to vaccines or other drugs in the past
7. Treatment with any vaccine, including influenza vaccine, within 4 months prior to planned first study drug dose or planned treatment with any vaccine up to 4 months after the last injection with study drug.
8. Participation in other clinical trials with a new chemical entity within the previous 3 months
9. Inability or unwillingness to comply with the provisions of this protocol
10. A history of alcohol or drug abuse
11. A significant illness other than diabetes within 2 weeks prior to first dosing
12. Known human immunodeficiency virus (HIV) or hepatitis
13. Females who are lactating or pregnant (the possibility of pregnancy must be excluded by urine βHCG on-site within 24 hours prior to the GAD-alum treatment)
14. Males or females not willing to use adequate contraception until 1 year after the last GAD-alum treatment
15. Presence of associated serious disease or condition, including active skin infections that preclude subcutaneous injection, which in the opinion of the investigator makes the patient non-eligible for the study
16. Deemed by the investigator not being able to follow instructions and/or follow the study protocol

### 5.3 Recruitment and screening

Eligible subjects will have the study explained to them, and will receive the written patient information. After having had the time to review the nature of the study, they will have the opportunity to ask questions to the investigational team. If, after this, the subjects agree to participate, they will personally sign and date the written informed consent form. The patients will then receive a copy of the signed and dated patient information/informed consent form.

### 5.4 Patient withdrawal

In accordance with the Declaration of Helsinki, the investigator must explain to the patient that they have the right to withdraw from the study at any time, and that this will in no way prejudice their future treatment. However, unless safety issues occur, we plan to follow the patients for the entire duration of the study in order to analyse efficacy and safety variables also for those patients withdrawing from the study. The reason for any kind of withdrawal must be recorded on the appropriate CRF.

There will be different categories for withdrawals from the study: Complete withdrawal (i.e. stopping investigational product and also continued efficacy and safety evaluations)

Standard reasons for withdrawing from further participation in the study and from the follow-up visits (and <e.g. blood tests>) may be:
- Patient's decision (withdrawal of consent to participate)
- Patient lost to follow-up
- Standard reasons from withdrawing from taking further investigational product, but continuing follow-up visits and safety evaluations may be:
- Unacceptable adverse events
- Patient request
- Investigator's discretion
- Patient lost to follow-up/non-attendance
- Intercurrent illness
- The patient becomes pregnant

Thus intra lymph node GAD-alum should not be given to the patient if the patient after inclusion in the study has got
- brain damage, epilepsia, head trauma, neurological disease
- any active, serious hormonal disease other than Type 1 diabetes
- other severe autoimmune disease( except celiac disease which is accepted for inclusion)
- immune-suppressive treatment
- cancer, cancer treatment
- any other diabetes drugs other than insulin
- any vaccination
- drug/alcohol abuse
or if the patient has
- become pregnant or is no longer willing to use safe contraceptives during the study

However, whenever a patient is withdrawn from a study, or for whatever reason is not coming to any further visits, a final study evaluation must be completed for that patient (visit <>) - stating the reason(s) why the patient was withdrawn from the study. All documentation concerning the patient must be as complete as possible. Withdrawals due to non-attendance must be followed up by the investigator to obtain the reason for non-attendance. Withdrawals due to intercurrent illnesses or adverse events must be fully documented in the case record form, with the addition of supplementary information if available and/or appropriate.

### 6. Treatment procedures

### 6.1 Study Design and Treatment

The trial is singlecenter, open-label, pilot study in GADA positive T1D patients of either gender, 18.00 to 29.99 years old, diagnosed with T1D within 6 months at time of screening (Visit 1) and fasting Cpeptide levels equal to or above 0.12 nmol/L. In total, approximately 5 patients will be recruited at one site in Linköping, Sweden. The patients will be assessed for eligibility at the screening visit (Visit 1) 10 to 21 days prior to the start of treatment. The screened patients will be assigned a sequential screening number and this screening number will be used as patient identification throughout the study.

Patients who qualify for inclusion in the study will then be enrolled in the study to receive investigational study drug at the subsequent visits according to table 1 below. The patients will be followed for a total study period of 30 months which includes 8 visits to the site.

See table 1 below for an overview of the study visits.

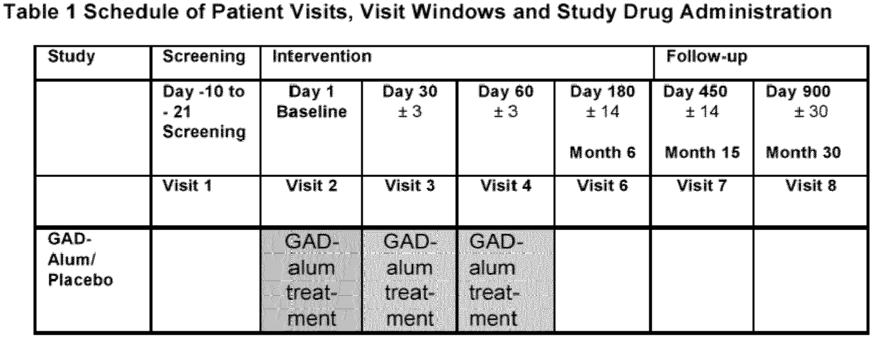

According to the present invention, further administrations of GAD-alum may be performed at visit 7 and optionally 8, as described above.

### 6.2 Assessments and procedures

1. Standard insulin treatment, education and psychosocial support for newly diagnosed Type 1 diabetes patients.
2. Normalization of fluid, electrolyte and acid-base balance.
3. Thereafter information about the study.
4. When the patients have given their informed consent, at the latest 120 days after diagnosis, screening is performed with a fasting venous sample from patients who are eligible according to other criteria than C-peptide and GADA concentrations (Visit 1).
5. At Baseline (Visit 2), 6, 15 and 30 months, assessment of residual endogenous insulin secretion by MMTT. HbA1c, safety (haematology and chemistry), autoantibody titres (GAD65, IA-2), immunology, are followed by blood samples at every study visit. Exogenous insulin dose/24 hours, Aes and concomitant medication is registered at every study visit.
6. Self-reported hypoglycaemia (defined as needing help from others and/or seizures and/or unconscious) registered at every study visit.
7. Any symptoms or signs of other medical problem should be treated at the discretion of the clinical doctor.

Examinations will be performed according to Table 2 in Section 7 below, and in the order outlined in the case report form (CRF).

### 6.2.1 All Visits, Visit 1 through 7

Note that the patient should attend all study visits in the morning following an overnight fast (> 10 hours, water allowed). For patients with evidence of an infection (including fever), the complete visit should be postponed for 5 days or until the patient has recovered.

### 6.2.2 Administrations of GAD-Alum, Visits 2, 3 and 4

After administration, the patient shall remain in the vicinity of the study site for the next hour, and the administration site will be examined by investigator/study nurse 1 hour post injection.

### 6.2.3 Mixed Meal Tolerance Test (MMTT), Visits 2, 5, 6 and 7

- The MMTT must be performed according to the instructions in the CRF. The patient should:
- Come to the study site following an overnight fast (>10hr), i.e. the patient may not eat but is permitted to drink water
- Not take short acting/direct acting insulin within 6 hours before the MMTT. The patient is allowed to take base-insulin day/night before, but not in the morning before the MMTT.
- Patients with CSII (insulin pump) must continue with their basal dose insulin, but not add bolus dose during the last 6 hours before the MMTT
- Have a fasting plasma glucose level in the range defined by 4-12 mmol/L on the patient's home blood glucose meter in the morning of the test If the patient does not fulfill all of the above criteria, the MMTT should be rescheduled and the patient should return to the study site within 5 days if possible.

If for safety reasons, subjects need to eat or take insulin, the visit should also be rescheduled.

### 6.3 Laboratory tests and examinations:

1. Immunological tests:
   a. Autoantibodies (Anti-GAD65, Anti-Insulin, Anti-IA-2, ZnT8)
   b. Relevant cytokines and chemokines are determined ( see below)
   c. T-cells are classified and studied (see below)
2. Genetics:
   a. HLA determinations is done and genes related to diabetes development
   b. Array studies to elucidate the importance of diabetes-related genes
3. Virus assays:
   a. Genetic, immunological and microbiological tests may be used.
4. Diabetes status:
   a. HbA1c
   b. Fasting glucose and fasting C-peptide
   c. Meal stimulated glucose and C-peptide
5. Blood sampling for safety:
   a. Hematology
   b. Chemistry

### 6.4 Medical history

A complete review of the subject's past medical history will be undertaken by the investigator and documented on the Medical History CRF.

All pre-existing conditions/diseases will be reported on the Medical History CRF page at the screening visit (Visit 1).

The subject's Type 1 diabetes diagnosis date and family history of Type 1 diabeteswill also be documented.

### 6.5 Physical Examination Including Neurological Examination

At the screening visit (Visit 1) the patient will undergo a general physical examination and a neurological examination and any findings will be reported as pre-existing conditions on the Medical History CRF page.

During the subsequent study visits the patient will be examined for any new medical conditions or worsening of the pre-existing ones. Any change in pre-existing conditions or new conditions must be entered on the AE page in the CRF and any medication given on the concomitant medication pages.

The patients will, in addition to the limited physical examination by the physician, undergo a standardized clinical neurological examination at screening, 0, 6, 15 and 30 months. The neurological tests are performed in order to detect possible mild signs of neuromuscular disease such as disturbance of strength, balance, and coordination.

The neurological examination includes:
- Extremity reflexes
- Romberg (balance and coordination)
- Walk on a line, 2 meters (balance and coordination)
- Standing on 1 leg, left and right, 15 seconds per leg (balance and coordination)
- Finger-nose (coordination)
- Mimic (cranial nerves)
- Babinski reflex (central function)
- Muscle strength (shake hands) biceps, triceps, distal extensors, and flexors

These examinations may also be repeated between scheduled visits at the discretion of the investigator. Screening for neurological disease with electroencephalogram (EEG) is not included due to low sensitivity and specificity. However, if any signs of neurological dysfunction are detected, the patient should be referred to a neurologist for further evaluation.

### 6.7 Concomitant Medication

Any concomitant medication used during the study, whether considered relevant for the study or not by the investigator must be reported on the concomitant medication log of the CRF. Please also see section 8.5, below.

### 7. Scheduling of procedures

**Table 1 Pilot DIAGNODE-1 Study, Schedule of Study Events, T1D patients**

| **Study Period** | **Screening** | **Intervention** | | | | **Follow-up** | |
|---|---|---|---|---|---|---|---|
| **Visit number** | **1** | **2** | **3** | **4** | **5** | **6** | **7** |
| Time (Day/Week/Month) | D -10 to - 21 | D1 Baseline | D30 ± 3 | D60 ± 3 | M6 (D180 ± 14) | M 15 (D450 ± 14) | M 30 (D900 ± 30) |
| Informed Consent | X | | | | | | |
| GAD-alum (Diamyd) treatment | | X | X | X | | | |
| Medical History | X | | | | | | |
| Physical Examination | X | X | X | X | X | X | X |
| Neurological Assessment | X | X | X | X | X | X | X |
| Concomitant Medication | X | | | | X | X | X |
| Height | X | X | X | X | X | | |
| Weight, BMI | X | X | X | X | | X | X |
| Urine pregnancy test (females) | | X | X | X | | | |
| Injection site inspection ^{a} | | X | X | X | | | |
| Insulin dose | X | X | X | X | X | X | X |
| Self Reported Hypoglycemia ^{b} | | X | X | X | X | X | X |
| Adverse Events | | X | X | X | X | X | X |
| Blood sampling for safety: | | | | | | | |
| * *Hematology* | X | X | X | X | X | X | X |
| * *Chemistry* | X | X | X | X | X | X | X |
| Auto-antibodies: | | | | | | | |
| * *GADA* | X | X | X | X | X | X | X |
| * *diabetes-related autoantibodies* | X | X | X | X | X | X | X |
| Blood sampling for Diabetes status: | | | | | | | |
| * *HbA1c* | X | X | X | X | X | X | X |
| * *Fasting glucose*/*C-peptide* | X | X | X | X | X | X | X |
| * *MMTT glucose*/ *C-peptide* | | X | | | X | X | X |
| Blood sampling for, genetics, immunology | | X | X | X | X | X | X |
| Vitamin D in serum | X | | | | | | |
| Blood sampling for biobank | X | X | X | X | X | X | X |

| | | | | | | | |
|---|---|---|---|---|---|---|---|
| ^{a} inspection of injection site before and 60 minutes after injection by investigator or nurse ^{b} Hypoglycemia defined as needing help from others and/or seizures and/or unconscious | | | | | | | |

According to the present invention, further administrations of GAD-alum may be performed at visit 7 and optionally 8, as described above.

### 7.1 Visits

The first visit, the screening visit (Visit 1) should be performed 10 to 21 days before planned Visit 2 (Baseline). Visit 3 and 4 should then be scheduled with a visit window of ±3 days (second and third administration of GAD-Alum) and of ±14 days for Visit 5, 6 and ±30 days for Visit 7. Please note that all visits must be calculated from the baseline visit (Visit 2) according to the visit schedule. Please also note that the visits must be performed within the visit windows to comply with the study protocol.

Please see table 1 and 2 above, for schedule of patient visits, visit windows and study drug administration.

### 8. Study medication

### 8.1 Study medication

The following medication supplies will be used in the study: Study medication: GAD-Alum (Diamyd), 4 µg × 3 (given three times with one month interval) IMP supplier: Diamyd Medical AB, Stockholm, Sweden.

### 8.2 Supply

GAD-alum (Diamyd) a formulation, supplied by Diamyd Medical. It will be supplied as pre-packed medication from Diamyd Medical to local pharmacy. All dosing will take place in the hospital, and handled only by trained and authorised study personnel. The study medication will be labelled with information according to local regulation. GAD-alum will be stored in a refrigerator at 2-8 ºC in a secure area (e.g. a locked cabinet or drug storage room), protected from unintended use. All study medication will be labelled with information according to local regulations.

### 8.3 Dosage and administration

GAD-Alum: 4 µg given into lymph node in the inguinal area (by help of ultra sound technique) three times with one month interval

### 8.4 Duration of treatment

See 8.3

### 8.5 Concomitant medication

No systemic immune modulating medication, and no other diabetes medication other than insulin, whether marketed or not, are allowed.

### 9. Response variables and outcomes

### 9.1 Exploratory assessment of efficacy

### 9.1.1. Efficacy variables

As this is a pilot Phase I study there is no primary efficacy endpoint, but we will still follow
- Change in C-peptide fasting and C-peptide (90 minute value and AUCmean 0-120 min) during an MMTT from baseline to month 6, 15 resp to month 30.
- Th2-deviation of cell-mediated immune response seen e.g. as increased ratio of IL-5, 10, 13 in comparison with IFN-gamma, TNF-alfa, IL-1beta, IL-17, and increase of T-regulatory cells. Change between baseline and subsequent visits.
- Inflammatory markers e.g. TNF-alfa, IL-1 beta, IL-2, IL-17. Change between baseline and subsequent visits.
- Hemoglobin A1c (HbA1c), change between baseline and subsequent visits
- Exogenous insulin dose per kg body weight and 24 hours, change between baseline and subsequent visits

### 10. Statistical methodology and data management

### 10.1 Study design

The DIAGNODE-1 study is an open-label pilot Phase I intervention study

Study Participants: Newly diagnosed classic type 1 diabetes patients: N=5. Age 18-29.99 years. Recruited from one Endocrinology clinic in Sweden

### 10.2 Estimation of sample size

Power analysis: No formal power analyses are done for this pilot study.

### 10.3 Statistical analysis plan

In brief the following analyses are planned: All continuous variables will have the following descriptive statistics displayed: number of observations (n), mean value, standard deviation, minimum, median, and maximum. All variables of a categorical nature will be displayed with frequencies and percentages. The tabulation of the descriptive statistics will be split by visit. Where appropriate, baseline (screening) descriptive statistics will also be included.

### Demographic and other baseline characteristics

Demographics and baseline characteristics will be presented using descriptive statistics (summary tables).

### Safety variables and Efficacy data

The AE/SAE data will be presented using a standardized tabulation of the frequency and incidence rate of all observed AEs/SAEs. The frequencies and incidence rates are calculated on a per patient basis. Adverse events will be summarized by body system, causality, and severity. Other safety data will be presented by descriptive statistics.

Efficacy data regarding C-peptide and immune system as well as Adverse events and other safety data will be summarized descriptively.

After 6 months analysis of the safety data. (The results will be used for design of a Phase II DIAGNODE trial).

### 10.4 Study populations

### Intention-to-treat population

Patients will be included in the primary intention-to-treat population for analysis of efficacy if they receive at least 1 dose of all study drugs in that arm, and are assessed at a later visit.

### Per protocol population

In order to qualify for the stringent per protocol population, the subjects must have followed the study protocol without any major violations. Any examinations missed will be substituted with the last observation carried forward, but examinations from not more than 1 visit may be lost.

### Total population

Any patient who withdraws after having received at least one treatment (visit 2) will be included in the safety analysis (adverse events and safety parameters). Data for all patients will be listed, and a list of withdrawn patients, with all reasons for withdrawal, will be given.

### 10.5 Data collection / case report forms

Case report forms (CRFs) will be supplied for recording data from each patient. Since it is important to have proper data collection in a timely manner, the investigator or assigned designee shall complete the CRFs promptly. When the monitor requests additional data or clarification of data for the CRF, the request must be answered satisfactorily in due time.

It is the responsibility of the investigator to ensure that these case report forms are properly completed. The investigator will sign the designated signature pages to confirm that the case report form is accurate and complete.

To ensure legibility the CRFs should be completed in block capitals with a black or blue ballpoint pen (not pencil, felt-tip or fountain pen). Any corrections to the CRFs must be carried out by the investigator or his designate. A single stroke must be drawn through the original entry. The correction has to be dated and initialled. Incorrect entries must not be covered with correcting fluid, or obliterated, or made illegible in any way. Even if there are no changes from a previous examination, in the interests of completeness of data acquisition the questions, which are repeated in each section of the CRFs should be answered in full. A reasonable explanation must be given by the investigator for all missing data.

### 10.6 Data management

Data will be coded and entered into a computer database. The handling of data, including data quality control, will comply with regulatory guidelines (e.g., International Conference on Harmonization [ICH] and Good Clinical Practice [GCP]).

### 11. Regulatory and administrative procedures

Any regulatory requirements must have been met before starting the study. The Sponsor will apply for the regulatory approval to the appropriate authorities. Study sites, facilities, laboratories and all data (including source data) and documentation must be made available for inspection by the authorities.

### 11.2 Patient Information / Informed Consent

The investigator is responsible for giving the patients full and adequate verbal and written information about the nature, purpose, possible risk and benefit of the study. Patients must also be notified that they are free to withdraw from the study at any time. The patients should have reasonable time to read and understand the information before signing. The investigator is responsible for obtaining signed informed consent from all patients before including the patient in any study related procedures. A copy of the patient information and of the Informed Consent Form will be given to the patients.

### 11.4 Patient treatment plan

All patients will continue to receive standard care for Type 1 diabetes during the study. After the individual completion of the study, the patient will return to the standard treatment received prior to study participation.

## Claims

1. A method for assessing the efficacy of an immunotherapy administered to a patient, said immunotherapy comprising administration of GAD, comprising the following steps:
- measuring at least one of
• GADA IgG subclass distribution;
• GADA levels;
• Distribution of cytokines secreted from lymphocytes; and
• Lymphocyte proliferation in presence of GAD or CD3/CD28 beads;
in a first blood, plasma or serum sample obtained from said patient at a first point in time and in a second blood, plasma or serum sample obtained from said patient at a second, later, point in time;
- Comparing the so obtained measurements;
wherein an increased relative amount of IgG₃, and/or IgG₄, or decreased relative amount of IgG₁ in the GADA IgG subclass distribution; increased GADA levels; an increased relative amount of IL-13 and/or IL-5 or a decreased relative amount of IFNy and/or TNFα in the distribution of cytokines secreted from lymphocytes; and/or reduced lymphocyte proliferation in the presence of GAD or CD3/CD28 beads; as measured in the second sample as compared to as measured in the first sample, is indicative of an effective immunotherapy.

2. The method according to claim 1, wherein at least two, at least three, or four of
• GADA IgG subclass distribution;
• GADA levels;
• Distribution of cytokines secreted from lymphocytes; and
• Lymphocyte proliferation;
are measured in said first and second blood, plasma or serum sample.

3. The method according to claim 1 or 2, wherein the first sample is obtained before or at commencement of the immunotherapy, or 80-100, such as 90, days after commencement of the immunotherapy; and/or
wherein the second sample is obtained at 160-200 days, such as 180 days; or at 12, 15, 24, 30 or 36 months after commencement of the immunotherapy, or wherein the second sample is obtained 160-200 days, such as 180 days; or at 12, 15, 24, 30 or 36 months after the first sample is obtained.

4. The method according to any one of claims 1-3, wherein GADA IgG subclass distribution is measured in said first and second samples and the so obtained measurements are compared, and wherein an increased amount of IgG₄ relative to the amount of IgG₁ in the GADA IgG subclass distribution, as measured in the second sample as compared to as measured in the first sample, is indicative of an effective immunotherapy and/or
wherein GADA IgG subclass distribution is measured in said first and second samples and the so obtained measurements are compared, and wherein an increased relative amount of IgG₄ in the GADA IgG subclass distribution, as measured in the second sample as compared to as measured in the first sample, is indicative of an effective immunotherapy; and/or
wherein GADA IgG subclass distribution is measured in said first and second samples and the so obtained measurements are compared, and wherein a decreased relative amount of IgG₁ in the GADA IgG subclass distribution, as measured in the second sample as compared to as measured in the first sample, is indicative of an effective immunotherapy; and/or
wherein GADA levels are measured in said first and second samples and the so obtained measurements are compared, wherein increased GADA levels, as measured in the second sample as compared to as measured in the first sample, are indicative of an effective immunotherapy; and/or
wherein distribution of cytokines secreted from lymphocytes is measured in said first and second samples and the so obtained measurements are compared, wherein increased relative amount of IL-13 and/or IL-5, as measured in the second sample as compared to as measured in the first sample, is indicative of an effective immunotherapy and/or
wherein distribution of cytokines secreted from lymphocytes is measured in said first and second samples and the so obtained measurements are compared, wherein an increased amount of IL-13 relative to the amount of IFNy, as measured in the second sample as compared to as measured in the first sample, is indicative of an effective immunotherapy; and/or
wherein distribution of cytokines secreted from lymphocytes is measured in said first and second samples and the so obtained measurements are compared, wherein decreased relative amount of IFNy and/or TNFα, as measured in the second sample as compared to as measured in the first sample, is indicative of an effective immunotherapy; and/or
wherein lymphocyte proliferation in presence of GAD or CD3/CD28 beads is measured in said first and second samples and the so obtained measurements are compared, wherein reduced lymphocyte proliferation in the presence of GAD or CD3/CD28 beads, as measured in the second sample as compared to as measured in the first sample, is indicative of an effective immunotherapy.

5. The method according to any one of claims 1-4, wherein said immunotherapy comprises administration of GAD by means of intralymphatic injection, including injection into a lymph node, intradermal injection, subcutaneous injection, or oral administration.

6. GAD for use in a method for treatment or prevention of type 1 diabetes by means of immunotherapy, comprising the steps of
- administration of GAD to a subject;
- obtaining an assessment of the efficacy of the immunotherapy by a method according to any one of claims 1-5; and
- adjusting the dosage and/or administration route of GAD based on said assessment.

7. GAD for use according to claim 6, wherein if the comparison of the obtained measurements is not indicative of an effective immunotherapy, then the adjustment of the dosage of GAD includes a further administration of GAD by injection into a lymph node.

8. GAD for use according to claim 6, wherein said method comprises
- administration of vitamin D commencing at day 1 and continuing for 3 to 6 months;
- three administrations of GAD into a lymph node of the subject at days 30, 60 and 90, respectively;
- obtaining an assessment of the efficacy of the immunotherapy by a method according to any one of claims 1-5;
and wherein if the comparison of the obtained measurements is not indicative of an effective immunotherapy, then the adjustment of the dosage of GAD includes a fourth administration of GAD into a lymph node at a time between 12 and 18 months after day 1.

9. GAD for use according to any one of claims 6-8, wherein a fourth or further administration is performed if HbA1c and required insulin dose have gone up at 12 to 18 months as compared to levels observed at 5 to 6 months after first GAD-alum injection, and optionally if the IFN/IL-13 ratio has not decreased between the said observations.

10. GAD for use according to any one of claims 6-9, further comprising a further administration of GAD into a lymph node at 30 months after day 1.

11. GAD for use according to claim 10, wherein the further administration of GAD-alum is performed if HbA1c and/or required insulin dose for the subject have gone up at 30 months as compared to levels observed at 15 months after first GAD-alum injection, and optionally if the IFN/IL-13 ratio has not decreased between the said observations.

12. GAD for use according to any one of claims 6-11, the use comprising at least three administrations of GAD into a lymph node of the subject at days 30, 60 and 90, respectively, and wherein at least one further administration into a lymph node is performed if HbA1c levels and/or required insulin dose for the subject at a given time have increased as compared to levels observed 6-24 months prior to said given time; or
the use comprising at least three administrations of GAD into a lymph node of the subject at days 30, 60 and 90, respectively, and wherein at least one further administration into a lymph node is performed if the relative amount of IL-13 and/or IL-5 in the distribution of cytokines secreted from lymphocytes is unchanged or decreased at a given time as compared to levels observed 6-24 months prior to said given time; or
the use comprising at least three administrations of GAD into a lymph node of the subject at days 30, 60 and 90, respectively and wherein at least one further administration into a lymph node is performed if the ratio IFNy/IL-13 in the distribution of cytokines secreted from lymphocytes is unchanged or increased at a given time as compared to a the same ratio observed 6-24 months prior to said given time; or
the use comprising at least three administrations of GAD into a lymph node of the subject at days 30, 60 and 90, respectively, and wherein at least one further administration into a lymph node is performed if the ratio of IgG1/IgG4 in the population of GAD specific antibodies is unchanged or increased at a given time as compared to the same ratio observed 6-24 months prior to said given time.

13. GAD for use according to any one of claims 6-12, comprising daily administration of vitamin D commencing 0-90 days prior to a fourth or any further administration of GAD into a lymph node;and/or
wherein vitamin D is administered at a dose of 2000 IU per day; and/or
wherein vitamin D is administered for 4 months.

14. GAD for use according to any one of claims 6-13, wherein GAD is administered in the form of alum-formulated GAD.
